# EUROPÄISCHE PATENTSCHRIFT

(11) **EP 1 455 739 B1**
(45) Veröffentlichungstag und Bekanntmachung des Hinweises auf die Patenterteilung: **28.11.2007**
(21) Anmeldenummer: 02795144.1
(22) Anmeldetag: 10.12.2002
(51) Int. Cl.: A61K 8/81, A61K 8/87, A61Q 5/02, A61Q 5/06, A61Q 5/12, A61Q 19/00

(54) **KOSMETISCHES MITTEL ENTHALTEND WENIGSTENS EIN COPOLYMER MIT N-VINYLLACTAMEINHEITEN**
COSMETIC AGENT CONTAINING AT LEAST ONE COPOLYMER HAVING N-VINYLLACTAM UNITS
AGENT COSMETIQUE CONTENANT AU MOINS UN COPOLYMERE COMPORTANT DES UNITES N-VINYLLACTAME

(30) Priorität: 11.12.2001 DE 10160720
(43) Veröffentlichungstag der Anmeldung: 15.09.2004
(73) Patentinhaber: BASF Aktiengesellschaft, 67056 Ludwigshafen (DE)
(72) Erfinder: NGUYEN-KIM, Son, 69502 Hemsbach (DE); HÖSSEL, Peter, 67105 Schifferstadt (DE); SCHUNTER, Walter, 67251 Freinsheim (DE)
(74) Vertreter: Reitstötter - Kinzebach
(86) Internationale Anmeldenummer: PCT/EP2002/014015
(87) Internationale Veröffentlichungsnummer: WO 2003/053381

(56) Entgegenhaltungen:
- EP-A- 1 002 811
- EP-A- 1 110 536
- WO-A-00/68282
- DE-A- 2 924 663
- DE-A- 10 008 263
- US-A- 5 608 021

## Beschreibung

Die vorliegende Erfindung betrifft ein kosmetisches Mittel, welches wenigstens ein wasserlösliches oder wasserdispergierbares Copolymer enthält, das keine Siliciumatom-haltigen Gruppen enthält und das durch zweistufige radikalische Copolymerisation wenigstens eines N-Vinyllactams, wenigstens eines anionogenen Monomers, wenigstens eines davon verschiedenen offenkettigen Monomers und gegebenenfalls weiterer damit copolymerisierbarer α,β-ethylenisch ungesättigter Verbindungen in Gegenwart einer Polymerkomponente mit Wiederholungseinheiten, die Ethergruppen aufweisen oder die sich von Vinylalkohol ableiten, erhältlich ist.

Polymere finden in Kosmetik und Medizin vielfache Anwendung. In Seifen, Cremes und Lotionen beispielsweise dienen sie in der Regel als Formulierungsmittel, z.B. als Verdicker, Schaumstabilisator oder Wasserabsorbens oder auch dazu, die reizende Wirkung anderer Inhaltsstoffe abzumildern oder die dermale Applikation von Wirkstoffen zu verbessern. Ihre Aufgabe in der Haarkosmetik dagegen bestehen darin, die Eigenschaften des Haares zu beeinflussen, beispielsweise zu festigen.

Zur Festigung von Haarfrisuren werden beispielsweise Vinyllactam-Homo- und Copolymere und Carboxylatgruppen-haltige Polymere eingesetzt. Anforderungen an Haarfestigerharze sind zum Beispiel eine starke Festigung bei hoher Luftfeuchtigkeit, Elastizität, Auswaschbarkeit vom Haar, Verträglichkeit in der Formulierung und ein angenehmer Griff des damit behandelten Haares.

Schwierigkeiten bereitet oft die Bereitstellung von Produkten mit einem komplexen Eigenschaftsprofil. So besteht ein Bedarf an Polymeren für kosmetische Mittel, die zur Bildung im Wesentlichen glatter, klebfreier Filme befähigt sind, die dem Haar und der Haut einen angenehmen Griff verleihen und gleichzeitig eine gute Festigungswirkung bzw. Konditionierwirkung aufweisen. Zudem werden an kosmetische und pharmazeutische Produkte vom Verbraucher zunehmend ästhetische Anforderungen gestellt. So wird bei derartigen Produkten derzeit eine Bevorzugung von Formulierungen in Form von Gelen, insbesondere klaren Gelen, beobachtet. Bei der Formulierung klarer Gele für die Kosmetik ist die Verträglichkeit der kosmetischen Wirkstoffe mit der Gelgrundlage besonders wichtig. Bekannte kosmetische Gelformulierungen basieren beispielsweise auf Polyvinylformamid oder auf einer Kombination von Polyvinylpyrrolidon mit geeigneten Verdickern, auf Vinylpyrrolidon/Vinylacetat-Copolymeren oder auf Copolymeren von Vinylpyrrolidon mit kationogenen bzw. kationischen Comonomeren.

Wasserlösliche oder wasserdispergierbare Copolymere auf Basis eines N-Vinyllactams sind in EP 1 002 811 und DE 100 08 263 offenbart.

Die EP-A-0 257 444 beschreibt Terpolymere aus Vinylpyrrolidon, tert.-Butyl(meth)acrylat und (Meth)acrylsäure und deren Verwendung in Haarbehandlungsmitteln.

Die DE-A-37 16 380 beschreibt Mittel zur Behandlung der Haut oder Haare in Form eines Aerosolschaums, die als Schäumungsmittel mindestens einen partiell acetylierten Polyvinylalkohol sowie weitere Inhaltsstoffe, wie kationische, anionische oder amphotere Agentien, enthalten.

Die US 5,632,977 beschreibt Haarpflegemittel, die ein Polymer enthalten, welches N-Vinylformamid sowie weitere Monomere, z.B. Monomere mit Amingruppen und Lactame, wie N-Vinylpyrrolidon einpolymerisiert enthält.

Die JP-A-0525 5057 beschreibt ein Hautschutzmittel, das ein Gemisch aus Polyvinylalkohol und Polyvinylpyrrolidon enthält.

Keines der zuvor genannten Dokumente beschreibt die Polymerisation von radikalisch polymerisierbaren ungesättigten Verbindungen (Monomeren) in Gegenwart vorgefertigter Polymere, die ihrerseits keine Kohlenstoff-Kohlenstoff-Doppelbindungen aufweisen.

Die DE-A-44 34 986 beschreibt ein Verfahren zur Herstellung wässriger Lösungen von Poly(N-Vinyl-ε-caprolactam) durch Polymerisation in Gegenwart eines polymeren Schutzkolloids. Dabei kann es sich beispielsweise um Polyvinylalkohol, teilverseiftes Polyvinylacetat, Polyalkylvinylether und Mischungen davon handeln. Ganz allgemein und ohne Beleg durch ein Anwendungsbeispiel wird eine Eignung der wässrigen Lösungen von Poly(N-Vinyl-ε-caprolactam) für kosmetische Zubereitungen, beispielsweise Haarlack, Haarspray und hautkosmetische Zubereitungen beschrieben.

Die JP-A-0618 4251 beschreibt Pfropfcopolymere von N-Vinylacetamid, gegebenenfalls in Kombination mit weiteren Vinylmonomeren, auf Polyvinylalkohol. Diese Pfropfcopolymere eignen sich für wasserlösliche Verpackungen.

Die WO 99/04750 beschreibt ein Verfahren zur Herstellung von Polymeren durch radikalische Polymerisation ethylenisch ungesättigter Monomere in Gegenwart von Polyalkylenoxid-haltigen Siliconderivaten.

Die DE-A-44 09 903 beschreibt N-Vinyleinheiten enthaltende Pfropfpolymerisate, Verfahren zu ihrer Herstellung und ihre Verwendung. Dabei werden auf eine Pfropfgrundlage, welche ein Polymerisat ist, das jeweils mindestens 5 Gew.-% Einheiten der Formeln 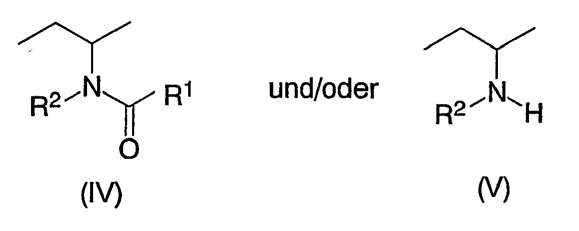 enthält, wobei R¹, R² = H oder C₁-C₆-Alkyl bedeuten, monoethylenisch ungesättigte Monomere aufgepfropft. Als monoethylenisch ungesättigte Monomere kommen alle ethylenisch ungesättigten Monomere in Frage, deren Polymerisation durch die Amingruppen in freier oder in Salzform nicht inhibiert wird, wie z.B. monoethylenisch ungesättigte Mono- und Dicarbonsäuren, deren Salze und Ester mit C₁-C₃₀-Alkoholen. Eine Eignung dieser Pfropfcopolymere als Wirkstoff in kosmetischen Formulierungen wird nicht beschrieben.

Die WO 96/34903 beschreibt N-Vinyleinheiten enthaltende Pfropfpolymerisate, Verfahren zu ihrer Herstellung und ihre Verwendung. Dabei werden auf eine Polyalkylenoxid-Pfropfgrundlage N-Vinylcarbonsäureamide und gegebenenfalls weitere Comonomere aufgepfropft und das resultierende Polymerisat anschließend zumindest teilweise verseift. Eine Eignung dieser Pfropfcopolymere als Wirkstoff in kosmetischen Formulierungen wird nicht beschrieben.

Die DE-A-29 24 663 beschreibt ein Verfahren zur Herstellung wässriger Dispersionen, bei dem man ein zur Bildung wasserlöslicher Polymere befähigtes Monomer a) in Gegenwart eines davon verschiedenen wasserlöslichen Polymers b) polymerisiert. Als Monomere a) werden (Meth)acrylamide, (Meth)acrylsäure, deren Halogenderivate und Salze sowie Ester der (Meth)acrylsäure mit Aminoalkanolen eingesetzt. Das Polymer b) weist wenigstens eine hydrophile Struktureinheit auf und ist beispielsweise ausgewählt unter Polyalkylenglycolen, Polyvinylalkohol, etc. Neben einer Vielzahl möglicher Verwendungen dieser Dispersionen wird ganz allgemein und ohne Beleg durch ein Ausführungsbeispiel auch ein Einsatz als Zusatz für Arzneimittel oder Kosmetika beschrieben.

Die unveröffentlichte deutsche Patentanmeldung P 100 412 11.4 beschreibt die Verwendung von Pfropfcopolymerisaten, die erhältlich sind durch radikalische Pfropfcopolymerisation von mindestens einer offenkettigen N-Vinylamid-Verbindung und gegebenenfalls wenigstens eines weiteren damit copolymerisierbaren Monomeren auf eine polymere Pfropfgrundlage, für kosmetische Anwendungen.

Der vorliegenden Erfindung liegt die Aufgabe zugrunde, kosmetische Mittel mit guten anwendungstechnischen Eigenschaften zur Verfügung zustellen. Diese sollen dem damit behandelten Haar bzw. der Haut einen angenehmen Griff verleihen und gleichzeitig eine gute Konditionierwirkung bzw. Festigungswirkung aufweisen. Vorzugsweise sollen sie sich zur Herstellung von Produkten in Form von Gelen eignen.

Überraschenderweise wurde nun gefunden, dass diese Aufgabe durch ein kosmetisches Mittel gelöst wird, das wenigstens ein Copolymer enthält, das keine Siliciumatom-haltigen Gruppen enthält und das durch zweistufige radikalische Copolymerisation wenigstens eines N-Vinyllactams, wenigstens eines anionogenen Monomers und wenigstens eines davon verschiedenen weiteren Monomers in Gegenwart einer wasserlöslichen Polymerkomponente, die im wesentlichen keine Kohlenstoff-Kohlenstoff-Doppelbindungen enthält, erhältlich ist.

Gegenstand der vorliegenden Erfindung ist daher ein kosmetisches oder pharmazeutisches Mittel enthaltend in einem kosmetisch akzeptablen Träger wenigstens ein wasserlösliches oder wasserdispergierbares Copolymer A), das keine Siliciumatom-haltigen Gruppen enthält und das durch zweistufige radikalische Copolymerisation eines Monomergemisches aus
a) 20 bis 99,9 Gew.-%, bezogen auf das Gesamtgewicht der Komponenten a) bis d), wenigstens eines N-Vinyllactams,
b) 0,1 bis 30 Gew.-%, bezogen auf das Gesamtgewicht der Komponenten a) bis d), wenigstens einer Verbindung mit einer radikalisch polymerisierbaren, α,β-ethylenisch ungesättigten Doppelbindung und mindestens einer anionogenen Gruppe pro Molekül,
c) 0,1 bis 65 Gew.-%, bezogen auf das Gesamtgewicht der Komponenten a) bis d), wenigstens einer radikalisch polymerisierbaren Verbindung, die ausgewählt ist unter
   c1) offenkettigen, α,β-ethylenisch ungesättigten Verbindungen der allgemeinen Formel I 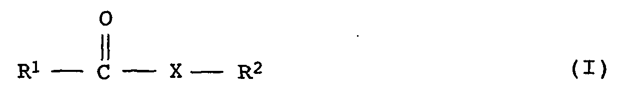 wobei
      einer der Reste R¹ oder R² für eine Gruppe der Formel CH₂=CR³- mit R³ = H oder C₁-C₄-Alkyl steht und der andere für H, Alkyl, Hydroxyalkyl, Aminoalkyl, den N-Alkyl- und N,N-Dialkyl-Derivaten davon oder einen Polyetherrest mit mindestens 5 Alkylenoxideinheiten steht, und
      X für O oder NR⁴ steht, wobei R⁴ für Wasserstoff, C₁-C₇-Alkyl oder einen Polyetherrest mit mindestens 5 Alkylenoxideinheiten steht,
   c2) Verbindungen mit einer radikalisch polymerisierbaren, α,β-ethylenisch ungesättigten Doppelbindung und mindestens einer anionischen oder kationischen Gruppe pro Molekül,
d) 0 bis 15 Gew.-%, bezogen auf das Gesamtgewicht der Komponenten a) bis d), wenigstens eines von a) bis c) verschiedenen copolymerisierbaren Monomeren
   in Gegenwart wenigstens einer wasserlöslichen Komponente e), die ausgewählt ist unter
e1) polyetherhaltigen Verbindungen, und/oder
e2) Polymeren, die mindestens 50 Gew.-% Wiederholungseinheiten aufweisen, die sich von Vinylalkohol ableiten,
erhältlich ist, wobei man:
i) die Monomere a) und gegebenenfalls wenigstens einen Teil der Monomere c) in Gegenwart wenigstens eines Teil der Komponente e) unter Erhalt eines ersten Polymerisats A1) polymerisiert, und
ii) die Monomere b) und falls vorhanden d) sowie die nicht bereits in Schritt i) eingesetzten Monomere c) und Verbindungen e) zugibt und unter Erhalt des Copolymers A) polymerisiert.

Im Rahmen der vorliegenden Erfindung umfassen die Ausdrücke C₁-C₇-Alkyl, C₁-C₄-Alkyl, C₈-C₃₀-Alkyl und C₂-C₂₂-Alkyl geradkettige und verzweigte Alkylgruppen. Geeignete kurzkettige Alkylgruppen sind z.B. geradkettige oder verzweigte C₁-C₇-Alkyl-, bevorzugt C₁-C₆-Alkyl- und besonders bevorzugt C₁-C₄-Alkylgruppen. Dazu zählen insbesondere Methyl, Ethyl, Propyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl etc.

Geeignete längerkettige C₈-C₃₀-Alkyl- bzw. C₈-C₃₀-Alkenylgruppen sind geradkettige und verzweigte Alkyl- bzw. Alkenylgruppen. Bevorzugt handelt es sich dabei um überwiegend lineare Alkylreste, wie sie auch in natürlichen oder synthetischen Fettsäuren und Fettalkoholen sowie Oxoalkoholen vorkommen, die gegebenenfalls zusätzlich einfach, zweifach oder mehrfach ungesättigt sein können. Bevorzugt sind C₈-C₂₂-Alkyl- bzw. -Alkenylgruppen. Dazu zählen z.B. n-Hexyl(en), 2- und 3-Methylpentyl(en), n-Heptyl(en), 2- und 3-Methylhexyl(en), n-Octyl(en), 2-, 3- und 4-Methylheptyl(en), 2- und 3-Ethylhexyl(en), n-Nonyl(en), n-Decyl(en), n-Undecyl(en), n-Dodecyl(en), n-Tridecyl(en), n-Tetradecyl(en), n-Pentadecyl(en), n-Hexadecyl(en), n-Heptadecyl(en), n-Octadecyl(en), n-Nonadecyl(en) etc.

Cycloalkyl steht vorzugsweise für C₅-C₈-Cycloalkyl, wie Cyclopentyl, Cyclohexyl, Cycloheptyl oder Cyclooctyl.

Heterocycloalkyl umfaßt gesättigte cycloaliphatische Reste mit im Allgemeinen 4 bis 8, vorzugsweise 5 bis 7 Ringatomen, in denen 1, 2 oder 3 der Ringatome für ein Heteroatom, ausgewählt unter Sauerstoff, Schwefel und gegebenenfalls substituiertem Stickstoff stehen. Dazu zählen beispielsweise Pyrrolidinyl, Piperidinyl, Imidazolidinyl, Pyrazolidinyl, Oxazolidinyl, Morpholidinyl, Thiazolidinyl, Isothiazolidinyl, Isoxazolidinyl, Piperazinyl, Tetrahydrothiophenyl, Tetrahydrofuranyl, Tetrahydropyranyl und Dioxanyl.

Aryl steht vorzugsweise für Phenyl, Tolyl, Xylyl oder Naphthyl.

Arylalkyl steht vorzugsweise für Benzyl oder Phenylethyl.

Im Folgenden werden Verbindungen, die sich von Acrylsäure und Methacrylsäure ableiten können teilweise verkürzt durch Einfügen der Silbe "(meth)" in die von der Acrylsäure abgeleitete Verbindung bezeichnet.

Die erfindungsgemäßen Mittel lassen sich unter Normalbedingungen (20°C) vorteilhaft als Gele formulieren. "Gelförmige Konsistenz" zeigen Mittel, die eine höhere Viskosität als eine Flüssigkeit aufweisen und die selbsttragend sind, d.h. die eine ihnen verliehene Form ohne formstabilisierende Umhüllung behalten. Im Gegensatz zu festen Formulierungen lassen sich gelförmige Formulierungen jedoch leicht unter Anwendung von Scherkräften deformieren. Die Viskosität der gelförmigen Mittel liegt vorzugsweise in einem Bereich von 600 bis 60000 mPas.

Unter wasserlöslichen Verbindungen (Monomeren, Polymeren) werden im Rahmen der vorliegenden Erfindung Verbindungen verstanden, die sich zu-mindestens 1 g/l bei 25°C in Wasser lösen. Unter wasserdispergierbaren Polymeren werden Polymere verstanden, die unter Anwendung von Scherkräften beispielsweise durch Rühren in dispergierbare Partikel zerfallen.

Die erfindungsgemäßen und zur Herstellung der erfindungsgemäßen kosmetischen Mittel eingesetzten Copolymere A) enthalten keine Siliciumatom-haltigen Gruppen.

Durch die radikalische Copolymerisation der Monomerkomponenten a), b) und c) sowie gegebenenfalls d) in Gegenwart wenigstens einer Verbindung der Komponente e) werden Copolymere A) mit vorteilhaften Eigenschaften erhalten. Dies kann beispielsweise aus einer zumindest teilweisen Pfropfung auf die Komponente e) als Pfropfgrundlage resultieren. Es sind jedoch auch andere Mechanismen als eine Pfropfung vorstellbar. Die Komponente A) umfasst ganz allgemein die Verfahrensprodukte der radikalischen Copolymerisation worunter z.B. reine Pfropfpolymerisate, Mischungen von Pfopfpolymerisaten mit ungepfropften Verbindungen der Komponente e), Homo- und Copolymerisate der Monomeren a) bis c) und gegebenenfalls d) sowie beliebige Mischungen verstanden werden.

Die Herstellung der erfindungsgemäßen und erfindungsgemäß in Kosmetika eingesetzten Copolymere A) erfolgt zweistufig, wobei im Schritt i) zwingend die N-Vinyllactame a) und im Schritt ii) zwingend die Monomere b) mit anionogenen (in der Regel sauren) funktionellen Gruppen eingesetzt werden. Die dabei resultierenden sequenziellen Copolymere eignen sich in besonders vorteilhafter Weise zur Herstellung von Gelformulierungen und weisen eine sehr gute Verträglichkeit mit einer Vielzahl in der Kosmetik eingesetzter Wirk- und Hilfsstoffe, speziell mit Polymeren auf.

Das Copolymer A) enthält 20 bis 99,9 Gew.-%, vorzugsweise 20 bis 90 Gew.-%, wenigstens eines N-Vinyllactams einpolymerisiert. Geeignet sind N-Vinyllactame, die zusätzlich zur Amidfunktion 3 bis 6 Ringkohlenstoffatome aufweisen und deren Derivate, die z.B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z.B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc. Bevorzugt werden N-Vinyllactame eingesetzt, die 3 bis 5 Ringkohlenstoffatome aufweisen. Besonders bevorzugt sind N-Vinylpyrrolidon und N-Vinylcaprolactam.

Die Monomere der Komponente a) werden erfindungsgemäß im 1. Schritt der Copolymerisation eingesetzt.

Der Einsatz wenigstens einer Komponente b) bei der Herstellung der Copolymere A) ist beispielsweise vorteilhaft um Copolymere A) zu erhalten, die sich zur Modifizierung der rheologischen Eigenschaften homogenphasiger oder heterogenphasiger Zusammensetzungen eignen. Unter "Modifizierung rheologischer Eigenschaften" wird unter anderem die Veränderung von Fließeigenschaften, die Erhöhung der Viskosität von Flüssigkeiten, die Verbesserung Thixotropie-Eigenschaften von Gelen, die Verfestigung von Gelen und Wachsen, etc. verstanden. Selbstverständlich können zur Einstellung der rheologischen Eigenschaften der erfindungsgemäßen kosmetischen Mittel auch übliche als Verdicker bekannte Substanzen eingesetzt werden.

Die anionogenen Gruppen der Verbindung b) sind vorzugsweise ausgewählt unter Carbonsäure-, Sulfonsäure- und Phosphonsäuregruppen.

Geeignete Verbindungen b) sind z.B. α,β-ethylenisch ungesättigte Mono- und/oder Dicarbonsäuren und deren Halbester und Anhydride, wie Acrylsäure, Methacrylsäure, Fumarsäure, Maleinsäure, Itaconsäure, Crotonsäure, Maleinsäureanhydrid, Monobutylmaleat, etc. Geeignete Verbindungen b) sind weiterhin Acrylamidoalkansulfonsäuren, wie 2-Acrylamido-2-methylpropansulfonsäure. Bevorzugt werden Acrylsäure und/oder Methacrylsäure eingesetzt.

Die Monomere der Komponente b) werden erfindungsgemäß im 2. Schritt der Copolymerisation eingesetzt. Eine etwaige Neutralisation der anionogenen Gruppe erfolgt erst nach der Polymerisation oder im Rahmen der Formulierung.

Die Komponente c) ist ausgewählt unter
c1) offenkettigen, α,β-ethylenisch ungesättigten Verbindungen der allgemeinen Formel I 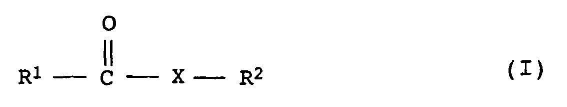 wobei
   einer der Reste R¹ oder R² für eine Gruppe der Formel CH₂=CR³- mit R³ = H oder C₁-C₄-Alkyl steht und der andere für H, Alkyl, Hydroxyalkyl, Aminoalkyl, den N-Alkyl- und N,N-Dialkyl-Derivaten davon oder einen Polyetherrest mit mindestens 5 Alkylenoxideinheiten steht, und
   X für O oder NR⁴ steht, wobei R⁴ für Wasserstoff, C₁-C₇-Alkyl oder einen Polyetherrest mit mindestens 5 Alkylenoxideinheiten steht,
c2) Verbindungen mit einer radikalisch polymerisierbaren, α,β-ethylenisch ungesättigten Doppelbindung und mindestens einer anionischen oder kationischen Gruppe pro Molekül
und Mischungen davon.

Die Verbindungen c1) sind vorzugsweise ausgewählt unter N-Vinyl-amiden gesättigter Monocarbonsäuren, Amiden α,β-ethylenisch ungesättigter Monocarbonsäuren und deren N-Alkyl-, N-Polyalkylenglycol-, N,N-Dialkyl-, Aminoalkyl-, (N-Alkylamino)alkyl-, (N,N-Dialkylamino)alkyl- und N,N-Di(polyalkylenglycol)-Derivaten, Estern von Vinylalkohol mit Monocarbonsäuren, Alkyl(meth)acrylaten, Hydroxyalkyl(meth)acrylaten, Polyetheracrylaten und Mischungen davon. Geeignete Polyalkylenglycolreste leiten sich beispielsweise von Polyethylenglycolen, Polypropylenglycolen, Polytetrahydrofuranen und Alkylenoxidcopolymeren ab. Geeignete Alkylenoxide zur Herstellung von Alkylenoxidcopolymeren sind z.B. Ethylenoxid, Propylenoxid, Epichlorhydrin, 1,2- und 2,3-Butylenoxid. Die Alkylenoxidcopolymere können die Alkylenoxideinheiten statistisch verteilt oder in Form von Blöcken einpolymerisiert enthalten. Bevorzugt sind Ethylen-oxid/Propylenoxid-Copolymere.

Bevorzugte Monomere c1) sind primäre Amide α,β-ethylenisch ungesättigter Monocarbonsäuren, wie Acrylamid, Methacrylamid, Ethacrylamid, etc. Besonders bevorzugt wird Acrylamid eingesetzt.

Bevorzugte Monomere c1) sind weiterhin Amide α,β-ethylenisch ungesättigter Mono- und Dicarbonsäuren mit Mono- und Dialkylaminen, die 1 bis 30 Kohlenstoffatome, bevorzugt 1 bis 22 Kohlenstoffatome, pro Alkylrest aufweisen. Dazu zählen bevorzugt Amide mit kurzkettigen Mono- und Di-C₁-C₇-alkylaminen, wie N-Methyl(meth)acrylamid, N-Ethyl(meth)acrylamid, N-(n-Propyl)(meth)acrylamid, N-Isopropyl(meth)acrylamid, N-(tert.-Butyl)(meth)acrylamid, N,N-Dimethyl(meth)acrylamid, etc. Geeignete Monomere c1) sind weiterhin Amide, die am Amidstickstoff einen oder zwei Substituenten aufweisen, die ausgewählt sind unter C₈-C₃₀-Alkyl, C₈-C₃₀-Alkenyl, Cycloalkyl-C₂-C₂₂-alkyl, Cycloalkyl-C₂-C₂₂-alkenyl, Aryl-C₂-C₂₂-alkyl oder Aryl-C₂-C₂₂-alkenyl.

Dazu zählen beispielsweise n-Octyl(meth)acrylamid, 1,1,3,3-Tetramethylbutyl(meth)acrylamid, Ethylhexyl(meth)acrylamid, n-Nonyl(meth)acrylamid, n-Decyl(meth)acrylamid, n-Undecyl(meth)acrylamid, Tridecyl(meth)acrylamid, Myristyl(meth)acrylamid, Pentadecyl(meth)acrylamid, Palmityl(meth)acrylamid, Heptadecyl(meth)acrylamid, Nonadecyl(meth)acrylamid, Arachinyl(meth)acrylamid, Behenyl(meth)acrylamid, Lignocerenyl(meth)acrylamid, Cerotinyl(meth)acrylamid, Melissinyl(meth)acrylamid, Palmitoleinyl(meth)acrylamid, Oleyl(meth)acrylamid, Linolyl(meth)acrylamid, Linolenyl(meth)acrylamid, Stearyl(meth)acrylamid, Lauryl(meth)acrylamid und Mischungen davon. Geeignete Monomere c1) sind auch die N-Alkylamide, N,N'-Dialkylamide, N-Alkyldiamide und N,N'-Dialkyldiamide der Maleinsäure.

Weitere geeignete Verbindungen c1) sind die Ester der zuvor genannten, α,β-ethylenisch ungesättigten Mono- und Dicarbonsäuren mit Aminoalkoholen, vorzugsweise C₂-C₁₂-Aminoalkoholen, welche am Aminstickstoff C₁-C₈-dialkyliert sind. Dazu zählen z.B. N,N-Dimethylaminomethyl(meth)acrylat, N,N-Dimethylaminoethyl(meth)acrylat, N,N-Diethylaminoethyl(meth)acrylat, N,N-Dimethylaminopropyl(meth)acrylat, N,N-Diethylaminopropyl(meth)acrylat, N,N-Dimethylaminocyclohexyl(meth)acrylat etc. Bevorzugt werden N,N-Dimethylaminopropylacrylat und N,N-Dimethylaminopropyl(meth)acrylat eingesetzt.

Geeignete Monomere c1) sind weiterhin die Amide der zuvor genannten α,β-ethylenisch ungesättigten Mono- und Dicarbonsäuren mit Diaminen, die eine tertiäre und ein primäre oder sekundäre Aminogruppe aufweisen. Dazu zählen z.B. N-[2-(dimethylamino)ethyl]acrylamid, N-[2-(dimethylamino)ethyl]methacrylamid, N-[3-(dimethylamino)propyl]acrylamid, N-[3-(dimethylamino)propyl]methacrylamid, N-[4-(dimethylamino)butyl]acrylamid, N-[4-(dimethylamino)butyl]methacrylamid, N-[2-(diethylamino)ethyl]acrylamid, N-[4-(dimethylamino)cyclohexyl]acrylamid, N-[4-(dimethylamino)cyclohexyl]methacrylamid etc. Besonders bevorzugt wird N-[3-(dimethylamino)propyl]methacrylamid (DMAPMA) eingesetzt.

Als Monomere c1) geeignete N-Vinylamide sind beispielsweise N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid, N-Vinyl-butyramid und Mischungen davon. Bevorzugt wird N-Vinylformamid eingesetzt.

Als Monomere c1) geeignete Ester von Vinylalkohol mit Monocarbonsäuren sind beispielsweise Vinylformiat, Vinylacetat, Vinylpropionat, Vinyl-n-butyrat, Vinylstearat und Vinyllaurat. Bevorzugt wird Vinylacetat eingesetzt.

Bevorzugte Verbindungen der Komponente c1) sind weiterhin die hinreichend wasserlöslichen Ester α,β-ethylenisch ungesättigter Mono- oder Dicarbonsäuren mit C₁-C₃-Alkanolen. Dazu zählen beispielsweise Methyl(meth)acrylat, Methylethacrylat, Ethyl(meth)acrylat, n-Propylacrylat und Mischungen davon. Bevorzugt wird Ethylacrylat eingesetzt. Geeignet als Monomere c1) sind jedoch auch Ester α,β-ethylenisch ungesättigter Mono- oder Dicarbonsäuren mit C₄-C₃₀-Alkanolen, bevorzugt C₄-C₂₂-Alkanolen.

Dazu zählen vorzugsweise die Ester der Acrylsäure, Methacrylsäure oder Ethacrylsäure mit einem gesättigten oder einfach oder mehrfach ungesättigten C₈-C₁₈-Alkohol, wie Octyl(meth)acrylat, 2-Ethylhexyl(meth)acrylat, Nonyl(meth)acrylat, Decycl(meth)acrylat, Lauryl(meth)acrylat, Pentadecyl(meth)acrylat, Palmityl(meth)acrylat, Arrachinyl(meth)acrylat, Myristyl(meth)acrylat, Cetyl(meth)acrylat, Stearyl(meth)acrylat, Lignocerenyl(meth)acrylat, Cerotinyl(meth)acrylat, Melissinyl(meth)acrylat, Palmitoleinyl(meth)acrylat, Oleyl(meth)acrylat, Behenyl(meth)acrylat, Cyclohexylethyl(meth)acrylat, Cyclohexyl-tert.-butyl(meth)acrylat etc.

Geeignete Monomere c1) sind auch Polyetheracrylate, worunter im Rahmen dieser Erfindung allgemein Ester α,β-ethylenisch ungesättigter Mono- und Dicarbonsäuren mit Polyetherolen verstanden werden. Geeignete Polyetherole sind lineare oder verzweigte, endständige Hydroxylgruppen aufweisende Substanzen, die Etherbindungen enthalten. Im Allgemeinen weisen sie ein Molekulargewicht im Bereich von etwa 150 bis 20 000 auf. Geeignete Polyetherole sind die zuvor genannten Polyalkylenglycole. Bevorzugt sind Polyetheracrylate der allgemeinen Formel II 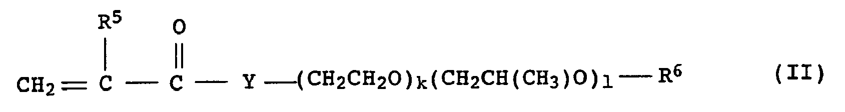 worin
die Reihenfolge der Alkylenoxideinheiten beliebig ist,
- k und 1: unabhängig voneinander für eine ganze Zahl von 0 bis 500 stehen, wobei die Summe aus k und 1 mindestens 5 beträgt,
- R⁵: für Wasserstoff oder C₁-C₈-Alkyl steht, und
- R⁶: für Wasserstoff oder C₁-C₆-Alkyl steht,
- Y: für O oder NR⁷ steht, wobei R⁷ für Wasserstoff, C₁-C₈-Alkyl oder C₅-C₈-Cycloalkyl steht.

Bevorzugt steht k für eine ganze Zahl von 1 bis 500, insbesondere 3 bis 250. Bevorzugt steht l für eine ganze Zahl von 0 bis 100.

Bevorzugt steht R⁵ für Wasserstoff, Methyl, Ethyl, n-Propyl, Isopropyl, n-Butyl, sec.-Butyl, tert.-Butyl, n-Pentyl oder n-Hexyl, insbesondere für Wasserstoff, Methyl oder Ethyl.

Vorzugsweise steht R⁶ in der Formel II für Wasserstoff, Methyl, Ethyl, n-Propyl, Isopropyl, n-Butyl, sec-Butyl, n-Pentyl oder n-Hexyl.

Vorzugsweise steht Y in der Formel II für O oder NH.

Geeignete Polyetheracrylate c1) sind z.B. die Polykondensationsprodukte der zuvor genannten α,β-ethylenisch ungesättigten Mono- und/oder Dicarbonsäuren und deren Säurechloriden, -amiden und Anhydriden mit Polyetherolen. Geeignete Polyetherole können leicht durch Umsetzung von Ethylenoxid, 1,2-Propylenoxid und/oder Epichlorhydrin mit einem Startermolekül, wie Wasser oder einem kurzkettigen Alkohol R⁶-OH hergestellt werden. Die Alkylenoxide können einzeln, alternierend nacheinander oder als Mischung eingesetzt werden. Die Polyetheracrylate c1) können allein oder in Mischungen zur Herstellung der erfindungsgemäß eingesetzten Polymere verwendet werden.

Als Monomere c1) geeignete Hydroxyalkyl(meth)acrylate sind vorzugsweise Hydroxy-C₁-C₄-alkyl(meth)acrylate, wie beispielsweise Hydroxyethyl(meth)acrylat, Hydroxy-n-propyl(meth)acrylat, etc.

Die anionischen Verbindungen c2) sind vorzugsweise ausgewählt unter den durch Neutralisation mit einer Base erhältlichen Salzen der zuvor beschriebenen anionogenen Verbindungen b). Geeignete Basen für die Neutralisation sind Alkalimetallbasen, wie Natronlauge, Kalilauge, Soda, Natriumhydrogencarbonat, Kaliumcarbonat oder Kaliumhydrogencarbonat und Erdalkalimetallbasen, wie Calciumhydroxid, Calciumoxid, Magnesiumhydroxid oder Magnesiumcarbonat sowie Ammoniak und Amine. Damit eine Verbindung der Komponente c2) zugeordnet wird, muß sie während der Polymerisation bereits in Salzform vorliegen. Dazu muß eine Neutralisation entsprechender Verbindungen b) bereits vor der Polymerisation erfolgen.

Kationische Verbindungen c2) sind durch teilweise oder vollständige Protonierung oder Quaternisierung der Amingruppen der zuvor genannten Amingruppen-haltigen Monomere erhältlich. Geeignete Säuren zur Protonierung sind z.B. Carbonsäuren, wie Milchsäure oder Mineralsäuren, wie Phosphorsäure, Schwefelsäure oder Salzsäure. Geeignete Alkylierungsmittel für die Quaternisierung sind beispielsweise C₁-C₄-Alkylhalogenide und -sulfate. Dazu zählen beispielsweise Ethylchlorid, Ethylbromid, Methylchlorid, Methylbromid, Dimethylsulfat und Diethylsulfat.

Die zuvor genannten Monomere c) können einzeln und in Form von Mischungen eingesetzt werden. Die Monomere c) können prinzipiell jeweils teilweise oder vollständig in der ersten und der zweiten Stufe der Copolymerisation eingesetzt werden. N-Vinylamide c) werden in der Regel in der ersten Stufe eingesetzt.

Geeignete Monomere d) sind z.B. Vinylaromaten, wie Styrol, α-Methylstyrol, o-Chlorstyrol und Vinyltoluole, Vinylhalogenide, wie Vinylchlorid, Vinylidenhalogenide, wie Vinylidenchlorid, Monoolefine, wie Ethylen und Propylen, nichtaromatische Kohlenwasserstoffe mit mindestens zwei konjugierten Doppelbindungen, wie Butadien, Isopren und Chloropren sowie Mischungen davon.

Die erfindungsgemäß eingesetzten Verbindungen der Komponente e) enthalten im Wesentlichen keine Kohlenstoff-Kohlenstoff-Doppelbindungen. Die Verbindungen der Komponente e) enthalten keine Siliciumatom-haltigen Gruppen.

Geeignete polyetherhaltige Verbindungen e1) sind im Allgemeinen wasserlösliche oder wasserdispergierbare, nichtionische Polymere, die Polyalkylenglycolgruppen aufweisen. Vorzugsweise beträgt der Anteil an Polyalkylenglycolgruppen mindestens 40 Gew.-%, bezogen auf das Gesamtgewicht der Verbindung e1). Als polyetherhaltige Verbindung e1) können beispielsweise die zuvor genannten Polyalkylenglycole, Polyester auf Basis von Polyalkylenglycolen sowie Polyetherurethane eingesetzt werden.

Je nach Art der zu ihrer Herstellung eingesetzten Monomerbausteine enthalten die polyetherhaltigen Verbindungen e1) folgende Struktureinheiten:
-(CH₂)₂-O-, -(CH₂)₃-O-, -(CH₂)₄-O-, -CH₂-CH(R⁸)-O-, worin
R⁸ für C₁-C₂₄-Alkyl, vorzugsweise C₁-C₄-Alkyl steht.

Die Verbindungen e1) können zusätzlich verbrückende Gruppen aufweisen, die beispielsweise ausgewählt sind unter:
-C(=O)-O-, -O-C(=O)-O-, -C(=O)-NR^{a}-, -O-C(=O)-NR^{a}-,
-NR^{b}-(C=O)-NR^{a}-
worin R^{a} und R^{b} unabhängig voneinander für Wasserstoff, C₁-C₃₀-Alkyl, vorzugsweise C₁-C₄-Alkyl oder Cycloalkyl stehen.

Bevorzugt werden als Polyether e1) Polymerisate der allgemeinen Formel III verwendet, mit einem Molekulargewicht >300 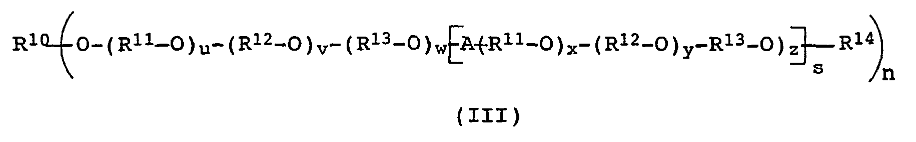 in der die Variablen unabhängig voneinander folgende Bedeutung haben:
- R¹⁰: Wasserstoff, C₁-C₂₄-Alkyl, R⁸-C(=O)-, R⁸-NH-C(=O)-, Polyalkoholrest;
- R¹⁴: Wasserstoff, C₁-C₂₄-Alkyl, R⁸-C(=O)-, R⁸-NH-C(=O)-;
- R¹¹ bis R¹³: -(CH₂)₂-, -(CH₂)₃-, -(CH₂)₄-, -CH₂-CH(R⁸)-, -CH₂-CHOR⁹-CH₂-;
- R⁸: C₁-C₂₄-Alkyl;
- R⁹: Wasserstoff, C₁-C₂₄-Alkyl, R⁸-C(=O)-, R⁸-NH-C(=O)-;
- A: -C(=O)-O, -C(=O)-B-C(=O)-O, -C(=O)-NH-B-NH-C(=O)-O;
- B: -(CH₂)ₜ-, gegebenenfalls substituiertes Cycloalkylen, Heterocycloalkylen oder Arylen;
- n: 1 bis 1000, bevorzugt 1 bis 200;
- s: 0 bis 1000, bevorzugt 0 bis 100;
- t: 2 bis 12, bevorzugt 1 bis 500;
- u: 1 bis 5000, bevorzugt 1 bis 500;
- v: 0 bis 5000, bevorzugt 1 bis 500;
- w: 0 bis 5000, bevorzugt 1 bis 500;
- x: 0 bis 5000, bevorzugt 1 bis 500;
- y: 0 bis 5000, bevorzugt 1 bis 500;
- z: 0 bis 5000, bevorzugt 1 bis 500.

Die endständigen primären Hydroxylgruppen der auf Basis von Polyalkylenoxiden hergestellten Polyether sowie die sekundären OH-Gruppen von Polyglycerin können dabei sowohl in ungeschützter Form frei vorliegen als auch mit Alkoholen einer Kettenlänge C₁-C₂₄ bzw. mit Carbonsäuren einer Kettenlänge C₁-C₂₄ verethert bzw. verestert werden oder mit Isocyanaten zu Urethanen umgesetzt werden. Bevorzugt werden Polyetherurethane eingesetzt.

Als bevorzugte Vertreter der oben genannten Alkylreste seien verzweigte oder unverzweigte C₁-C₁₂-, besonders bevorzugt C₁-C₆-Alkylketten genannt.

Das Molekulargewicht der Polyether liegt im Bereich größer 300 (nach Zahlenmittel), bevorzugt im Bereich von 300 bis 100000, besonders bevorzugt im Bereich von 500 bis 50000, ganz besonders bevorzugt im Bereich von 800 bis 40000.

Vorteilhafterweise verwendet man Homopolymerisate des Ethylenoxids oder Copolymerisate, mit einem Ethylenoxidanteil von 40 bis 99 Gew.-%. Für die bevorzugt einzusetzenden Ethylenoxidpolymerisate beträgt somit der Anteil an einpolymerisiertem Ethylenoxid 40 bis 100 mol-%. Als Comonomer für diese Copolymerisate kommen Propylenoxid, Butylenoxid und/oder Isobutylenoxid in Betracht. Geeignet sind beispielsweise Copolymerisate aus Ethylenoxid und Propylenoxid, Copolymerisate aus Ethylenoxid und Butylenoxid sowie Copolymerisate aus Ethylenoxid, Propylenoxid und mindestens einem Butylenoxid. Der Ethylenoxidanteil der Copolymerisate beträgt vorzugsweise 40 bis 99 mol-%, der Propylenoxidanteil 1 bis 60 mol-% und der Anteil an Butylenoxid in den Copolymerisaten 1 bis 30 mol-%. Neben geradkettigen können auch verzweigte Homo- oder Copolymerisate als polyetherhaltige Verbindungen e1) verwendet werden.

Verzweigte Polymerisate können hergestellt werden, indem man beispielsweise an Polyalkoholresten, z.B. an Pentaerythrit, Glycerin oder an Zuckeralkoholen wie D-Sorbit und D-Mannit aber auch an Polysaccharide wie Cellulose und Stärke, Ethylenoxid und gegebenenfalls noch Propylenoxid und/oder Butylenoxide anlagert. Die Alkylenoxid-Einheiten können im Polymerisat statistisch verteilt sein oder in Form von Blöcken vorliegen.

Es ist aber auch möglich, Polyester von Polyalkylenoxiden und aliphatischen oder aromatischen Dicarbonsäuren, z.B. Oxalsäure, Bernsteinsäure, Adipinsäure und Terephthalsäure mit Molmassen von 1500 bis 25000, wie z.B. beschrieben in EP-A-0 743 962, als polyetherhaltige Verbindung zu verwenden. Des weiteren können auch Polycarbonate durch Umsetzung von Polyalkylenoxiden mit Phosgen oder Carbonaten wie z.B. Diphenylcarbonat, sowie Polyurethane durch Umsetzung von Polyalkylenoxiden mit aliphatischen und aromatischen Diisocyanaten verwendet werden.

Nach einer bevorzugten Ausführungsform wird zur Herstellung der Copolymere A) eine Komponente e1) eingesetzt, die wenigstens ein Polyetherurethan umfasst.

Geeignete Polyetherurethane sind die Kondensationsprodukte von Polyetherpolyolen, wie Polyetherdiolen, mit Polyisocyanaten, wie Diisocyanaten. Geeignete Polyetherpolyole sind die zuvor genannten Polyalkylenglycole, die beispielsweise aus der Polymerisation von cyclischen Ethern, wie Tetrahydrofuran, oder aus der Umsetzung von einem oder mehreren Alkylenoxiden mit einem Startermolekül, das zwei oder mehr aktive Wasserstoffatome aufweist, erhältlich sind.

Geeignete Polyisocyanate sind ausgewählt unter Verbindungen mit 2 bis 5 Isocyanatgruppen, Isocyanatpräpolymeren mit einer mittleren Anzahl von 2 bis 5 Isocyanatgruppen, und Mischungen davon. Dazu zählen z.B. aliphatische, cycloaliphatische und aromatische Di-, Tri- und Polyisocyanate. Geeignete Diisocyanate sind z.B. Tetramethylendiisocyanat, Hexamethylendiisocyanat, 2,3,3-Trimethylhexamethylendiisocyanat, 1,4-Cyclohexylendiisocyanat, Isophorondiisocyanat, 1,4-Phenylendiisocyanat, 2,4- und 2,6-Toluylendiisocyanat und deren Isomerengemische (z.B. 80 % 2,4- und 20 % 2,6-Isomer), 1,5-Naphthylendiisocyanat, 2,4- und 4,4'-Diphenylmethandiisocyanat. Ein geeignetes Triisocyanat ist z.B. Triphenylmethan-4,4',4"-triisocyanat. Weiterhin geeignet sind Isocyanatpräpolymere und Polyisocyanate, die durch Addition der zuvor genannten Isocyanate an polyfunktionelle hydroxyl- oder amingruppenhaltige Verbindungen erhältlich sind. Weiterhin geeignet sind Polyisocyanate, die durch Biuret- oder Isocyanuratbildung entstehen. Bevorzugt werden Hexamethylendiisocyanat, trimerisiertes Hexamethylendiisocyanat, Isophorondiisocyanat, 2,4-Toluylendiisocyanat, 2,6-Toluylendiisocyanat, und Mischungen davon, eingesetzt.

Als Propfgrundlage eignen sich weiterhin Polymerisate e2), die mindestens 50 Gew.-% an Vinylalkoholeinheiten besitzen. Bevorzugt enthalten diese Polymerisate mindestens 70 Gew.-%, ganz besonders bevorzugt mindestens 80 Gew.-% Polyvinylalkoholeinheiten. Solche Polymerisate werden überlicherweise durch Polymerisation eines Vinylesters und anschließender zumindest teilweiser Alkoholyse, Aminolyse oder Hydrolyse hergestellt. Bevorzugt sind Vinylester linearer und verzweigter C₁-C₁₂-Carbonsäuren, ganz besonders bevorzugt ist Vinylacetat. Die Vinylester können selbstverständlich auch im Gemisch eingesetzt werden.

Als Comonomere des Vinylesters zur Synthese der Pfropfgrundlage e2) kommen beispielsweise N-Vinylcaprolactam, N-Vinylpyrrolidon, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage.

Die Herstellung der Pfropfgrundlage e2) erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200°C, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Zur Herstellung der Pfropfgrundlage e2) werden die Estergruppen der ursprünglichen Monomere und gegebenenfalls weiterer Monomere nach der Polymerisation durch Hydrolyse, Alkoholyse oder Aminolyse zumindest teilweise gespalten. Im nachfolgenden wird dieser Verfahrensschritt allgemein als Verseifung bezeichnet. Die Verseifung erfolgt in an sich bekannter Weise durch Zugabe einer Base oder Säure, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base bzw. Säure, von der Verseifungstemperatur, der Verseifungszeit und dem Wassergehalt der Lösung.

Besonders bevorzugte Propfgrundlagen e2) sind Polymerisate, die durch Homopolymerisation von Vinylacetat und anschließender zumindest teilweiser Hydrolyse, Alkoholyse oder Aminolyse hergestellt werden. Solche Polyvinylalkoholeinheiten enthaltenden Polymere sind unter dem Namen Mowiol® erhältlich.

Das erfindungsgemäße und in den erfindungsgemäßen Mitteln eingesetzte Copolymer A) ist vorzugsweise erhältlich durch radikalische Copolymerisation von
- 20 bis 90 Gew.-% wenigstens eines N-Vinyllactams a),
- 1 bis 20 Gew.-% wenigstens einer Verbindung b) und
- 5 bis 65 Gew.-% wenigstens einer Verbindung c)
in Gegenwart wenigstens einer Komponente e).

Besonders bevorzugt ist das Copolymerisat A) erhältlich durch radikalische Copolymerisation von
a) 20 bis 90 Gew.-% N-Vinylpyrrolidon und/oder N-Vinylcaprolactam,
b) 1 bis 20 Gew.-% (Meth)acrylsäure,
c) 5 bis 65 Gew.-% wenigstens einer Verbindung, die ausgewählt ist unter N-Vinylamiden gesättigter Monocarbonsäuren, Amiden α,β-ethylenisch ungesättigter Monocarbonsäuren, N,N-Dialkylaminoalkyl(meth)acrylaten, C₁-C₃-Alkyl(meth)acrylaten, Polyalkylenglycol(meth)acrylaten und Mischungen davon,
in Gegenwart wenigstens einer Komponente e).

Ein bevorzugtes Copolymerisat A) ist beispielsweise erhältlich durch radikalische Copolymerisation von
a) 25 bis 80 Gew.-% N-Vinylpyrrolidon und/oder N-Vinylcaprolactam,
b) 1 bis 10 Gew.-% (Meth)acrylsäure,
c1) 5 bis 45 Gew.-% Acrylamid,
c2) 0 bis 6 Gew.-% wenigstens eines Monomeren mit mindestens einer kationischen Gruppe pro Molekül, bevorzugt protonisiertes oder quaternisiertes Dimethylaminoethylmethacrylat,
in Gegenwart wenigstens einer Komponente e).

Das Gewichtsmengenverhältnis der Gesamtmenge der Komponenten a) bis d) zur Komponente e) beträgt vorzugsweise 100:1 bis 1:1, besonders bevorzugt 50:1 bis 3:1.

Die Herstellung der Copolymere A) erfolgt nach üblichen, dem Fachmann bekannten Verfahren, wie z.B. Lösungs-, Fällungs-, Emulsions-, umgekehrte Emulsions-, Suspensions- oder umgekehrte Suspensions-Polymerisation. Bevorzugt ist die Lösungspolymerisation.

### Schritt i)

Erfindungsgemäß wird im ersten Schritt der radikalischen Copolymerisation wenigstens ein N-Vinyllactam a), gegebenenfalls wenigstens ein Monomer c) in Gegenwart wenigstens eines Teils der Komponente e) unter Erhalt des ersten Polymerisats A1) polymerisiert.

Die eingesetzten Monomere können teilweise oder vollständig in der Reaktionszone vorgelegt werden. Vorzugsweise werden die Monomere nicht oder nur teilweise in der Reaktionszone vorgelegt und dieser im Übrigen nach Maßgabe ihres Verbrauchs kontinuierlich zugeführt. Die Zuführung mehrerer Monomere kann separat oder in Gemischen erfolgen.

Die Polymerisation in Schritt i) erfolgt in Gegenwart wenigstens eines Teils der Komponente e). Diese kann entweder vollständig zu Beginn der Polymerisation in der Reaktionszone vorgelegt oder teilweise oder vollständig im Verlauf der Polymerisation der Reaktionszone zugeführt werden.

Vorzugsweise erfolgt die Polymerisation in Schritt i) in Wasser als Lösungsmittel.

Die Polymerisationstemperatur in Schritt i) liegt vorzugsweise in einem Bereich von 30 bis 140°C, insbesondere 50 bis 90°C. Bevorzugt erfolgt die Polymerisation in Schritt i) bei einem pH-Wert von mindestens 6,0, besonders bevorzugt mindestens 6,5, insbesondere mindestens 7.

Das in Schritt i) erhaltene Polymerisat A1) kann vor der weiteren Umsetzung in Schritt ii) gewünschtenfalls isoliert und/oder nach üblichen, dem Fachmann bekannten Methoden gereinigt werden. Dazu zählt beispielsweise die Sprühtrocknung. Die erhaltenen Polymerisate A1) stellen neue Zwischenprodukte dar, die neben einem Einsatz in Schritt ii) des erfindungsgemäßen Verfahrens auch als Grundlage zur Herstellung weiterer Polymere (z.B. im Sinne einer Pfropfung) dienen können. Vorzugsweise wird das in Schritt i) erhaltene Reaktionsgemisch ohne Aufarbeitung im Folgeschritt ii) eingesetzt.

### Schritt ii)

Erfindungsgemäß wird im zweiten Schritt der radikalischen Copolymerisation das Präpolymer A1) mit wenigstens einem Monomer b) unter Erhalt des Copolymers A) umgesetzt. Gewünschtenfalls können auch in Schritt ii) Monomere c) als Comonomere eingesetzt werden. Die Monomere d), soweit eingesetzt, werden zwingend als Comonomere in Schritt ii) eingesetzt. Falls in Schritt ii) ein verbliebener Teil der Komponente e) eingesetzt wird, so kann dieser vollständig zu Beginn des zweiten Polymerisationsschrittes oder im Verlauf der Polymerisation der Reaktionszone zugeführt werden.

Die in Schritt ii) eingesetzten Monomere werden vorzugsweise nach Maßgabe ihres Verbrauchs der Reaktionszone zugeführt. Wasserlösliche Monomere können vorzugsweise als wässrige Lösung, weniger wasserlösliche oder wasserunlösliche beispielsweise als wässrig/alkoholische oder alkoholische Lösung eingesetzt werden.

Vorzugsweise erfolgt die Polymerisation in Schritt ii) in einem Wasser/C₁-C₄-Alkanol-Gemisch als Lösungsmittel. Dazu werden dem Reaktionsgemisch aus Schritt i) vorzugsweise bis zu 50 Gew.-% wenigstens eines Alkohols, bezogen auf die Gesamtlösungsmittelmenge, zugesetzt. Bevorzugt sind Wasser/Ethanol-Gemische.

Die Polymerisationstemperatur in Schritt ii) liegt vorzugsweise in einem Bereich von 40 bis 140°C. Vorzugsweise wird die Reaktionstemperatur nach Beendigung der Monomerzugabe auf mindestens 90°C, bevorzugt mindestens 100°C, zur Vervollständigung der Polymerisation, erhöht.

Vorzugsweise erfolgt die Polymerisation in Schritt ii) bei einem pH-Wert von weniger als 6,5, speziell höchstens 6.

Die Polymerisation erfolgt in beiden Schritten üblicherweise unter atmosphärischem Druck, sie kann jedoch auch unter vermindertem oder erhöhtem Druck ablaufen. Ein geeigneter Druckbereich liegt zwischen 1 und 5 bar.

Zur Herstellung der Polymerisate können die eingesetzten Monomere der Komponenten a) bis d) in Gegenwart der Komponente e) sowohl mit Hilfe von Radikale bildenden Initiatoren als auch durch Einwirkung energiereicher Strahlung, worunter auch die Einwirkung energiereicher Elektronen verstanden werden soll, polymerisiert werden.

Als Initiatoren für die radikalische Polymerisation können in beiden Schritten die hierfür üblichen Peroxo- und/oder Azo-Verbindungen eingesetzt werden, beispielsweise Alkali- oder Ammoniumperoxidisulfate, Diacetylperoxid, Dibenzoylperoxid, Succinylperoxid, Di-tert.-butylperoxid, tert.-Butylperbenzoat, tert.-Butylperpivalat, tert.-Butylperoxy-2-ethylhexanoat, tert.-Butylpermaleinat, Cumolhydroperoxid, Diisopropylperoxidicarbamat, Bis-(o-toluoyl)-peroxid, Didecanoylperoxid, Dioctanoylperoxid, Dilauroylperoxid, tert.-Butylperisobutyrat, tert.-Butylperacetat, Di-tert.-Amylperoxid, tert.-Butylhydroperoxid, Azo-bis-isobutyronitril, Azo-bis-(2-amidinopropan)dihydrochlorid oder 2,2'-Azo-bis-(2-methyl-butyronitril). Geeignet sind auch Initiatormischungen oder Redox-Initiator-Systeme, wie z.B. Ascorbinsäure/Eisen(II)sulfat/Natriumperoxodisulfat, tert.-Butylhydroperoxid/Natriumdisulfit, tert.-Butylhydroperoxid/Natriumhydroxymethansulfinat.

Bevorzugt werden wasserlösliche Initiatoren eingesetzt.

Die verwendeten Mengen an Initiator bzw. Initiatorgemischen bezogen auf eingesetzte Monomere liegen für beide Stufen gemeinsam im Allgemeinen zwischen 0,01 und 10 Gew.-%, vorzugsweise zwischen 0,1 und 5 Gew.-%.

Gegenstand der Erfindung ist auch ein Verfahren zur Herstellung wenigstens eines wasserlöslichen oder wasserdispergierbaren Copolymers A) durch radikalische Copolymerisation, wie zuvor beschrieben.

Copolymere A), die Säuregruppen enthalten, können teilweise neutralisiert werden, mit der Maßnahme, dass das resultierende Copolymer noch anionogene Gruppen entsprechend einem einpolymerisierten Anteil von 0,1 bis 30 Gew.-% der Komponente b) enthält. Geeignete Basen zur Neutralisation sind die zuvor zur Neutralisation der ionogenen Monomere b) genannten. Polymere mit Amingruppen können auch durch Umsetzung mit Säuren oder Quaternisierungsmitteln, z.B. mit Alkylierungsmitteln, wie C₁-C₄-Alkylhalogeniden oder -sulfaten in kationische Gruppen überführt werden. Geeignete Säuren und Alkylierungsmittel wurden zuvor genannt. In aller Regel weisen die erhaltenen Salze der Polymere eine bessere Wasserlöslichkeit oder Dispergierbarkeit in Wasser auf als die nicht neutralisierten oder quaternisierten Polymere.

Wird bei der Herstellung der Polymere ein organisches Lösungsmittel eingesetzt, so kann dieses durch übliche, dem Fachmann bekannte Verfahren, z.B. durch Destillation bei vermindertem Druck, entfernt werden. Zur Konservierung können den Copolymeren übliche Konservierungsmittel zugesetzt werden. Ein bevorzugtes Konservierungsmittel ist Ethanol.

Die Polymerlösungen und -dispersionen können durch verschiedene Trocknungsverfahren, wie z.B. Sprühtrocknung, Fluidized Spray Drying, Walzentrocknung oder Gefriertrocknung in Pulverform überführt werden. Bevorzugt wird die Sprühtrocknung eingesetzt. Die so erhaltenen Polymer-Trockenpulver lassen sich vorteilhafterweise durch Lösen bzw. Redispergieren in Wasser erneut in eine wässrige Lösung bzw. Dispersion überführen. Pulverförmige Copolymere haben den Vorteil einer besseren Lagerfähigkeit, einer einfacheren Transportmöglichkeit und zeigen in der Regel eine geringere Neigung für Keimbefall.

Gegenstand der Erfindung sind auch die Copolymere A).

Der kosmetisch akzeptable Träger ist vorzugsweise ausgewählt unter
i) Wasser,
ii) wassermischbaren organischen Lösungsmitteln, vorzugsweise C₁-C₄-Alkanolen,
iii) Treibgasen, vorzugsweise Propan/Butan-Gemischen und Dimethylether,
iv) Ölen, Fetten, Wachsen,
v) von iv) verschiedenen Estern von C₆-C₃₀-Monocarbonsäuren mit ein-, zwei- oder dreiwertigen Alkoholen,
vi) gesättigten acyclischen und cyclischen Kohlenwasserstoffen,
vii) Fettsäuren,
viii) Fettalkoholen
und Mischungen davon.

Vorzugsweise weisen die erfindungsgemäßen Mittel wenigstens einen hydrophilen kosmetisch oder pharmazeutisch akzeptablen Träger auf. Geeignete hydrophile Träger sind ausgewählt unter Wasser, 1-, 2- oder mehrwertigen Alkoholen mit vorzugsweise 1 bis 8 Kohlenstoffatomen, wie Ethanol, n-Propanol, iso-Propanol, Propylenglycol, Glycerin, Sorbit, etc.

Zur Herstellung von Aerosol-Formulierungen (Sprays) können die erfindungsgemäßen Mittel wenigstens ein Treibgas (Treibmittel) aufweisen. Dazu zählen beispielsweise Propan, n-Butan, n-Pentan, C₃-C₅-Alkangemische, Dimethylether, etc.

Die erfindungsgemäßen Mittel können als Träger auch z.B. eine Öl- bzw. Fettkomponente aufweisen, die ausgewählt ist unter: Kohlenwasserstoffen geringer Polarität, wie Mineralölen; linearen gesättigten Kohlenwasserstoffen, vorzugsweise mit mehr als 8 C-Atomen, wie Tetradecan, Hexadecan, Octadecan etc.; cyclischen Kohlenwasserstoffen, wie Decahydronaphthalin; verzweigten Kohlenwasserstoffen; tierischen und pflanzlichen Ölen; Wachsen; Wachsestern; Vaselin; Estern, bevorzugt Estern von Fettsäuren, wie z.B. die Ester von C₁-C₂₄-Monoalkoholen mit C₁-C₂₂-Monocarbonsäuren, wie Isopropylisostearat, n-Propylmyristat, iso-Propylmyristat, n-Propylpalmitat, iso-Propylpalmitat, Hexacosanylpalmitat, Octacosanylpalmitat, Triacontanylpalmitat, Dotriacontanylpalmitat, Tetratriacontanylpalmitat, Hexacosanylstearat, Octacosanylstearat, Triacontanylstearat, Dotriacontanylstearat, Tetratriacontanylstearat; Salicylaten, wie C₁-C₁₀-Salicylaten, z.B. Octylsalicylat; Benzoatestern, wie C₁₀-C₁₅-Alkylbenzoaten, Benzylbenzoat; anderen kosmetischen Estern, wie Fettsäuretriglyceriden, Propylenglykolmonolaurat, Polyethylenglykolmonolaurat, C₁₀-C₁₅-Alkyllactaten, etc. und Mischungen davon.

Geeignete Siliconöle sind z.B. lineare Polydimethylsiloxane, Poly(methylphenylsiloxane), cyclische Siloxane und Mischungen davon. Das zahlenmittlere Molekulargewicht der Polydimethylsiloxane und Poly(methylphenylsiloxane) liegt vorzugsweise in einem Bereich von etwa 1000 bis 150000 g/mol. Bevorzugte cyclische Siloxane weisen 4- bis 8-gliedrige Ringe auf. Geeignete cyclische Siloxane sind z.B. unter der Bezeichnung Cyclomethicon kommerziell erhältlich.

Bevorzugte Öl- bzw. Fettkomponenten sind ausgewählt unter Paraffin und Paraffinölen; Vaselin; natürlichen Fetten und Ölen, wie Castoröl, Sojaöl, Erdnussöl, Olivenöl, Sonnenblumenöl, Sesamöl, Avocadoöl, Kakaobutter, Mandelöl, Pfirsichkernöl, Ricinusöl, Lebertran, Schweineschmalz, Walrat, Spermacetöl, Spermöl, Weizenkeimöl, Macadamianussöl, Nachtkerzenöl, Jojobaöl; Fettalkoholen, wie Laurylalkohol, Myristylalkohol, Cetylalkohol, Stearylalkohol, Oleylalkohol, Cetylalkohol; Fettsäuren, wie Myristinsäure, Stearinsäure, Palmitinsäure, Ölsäure, Linolsäure, Linolensäure und davon verschiedenen gesättigten, ungesättigten und substituierten Fettsäuren; Wachsen, wie Bienenwachs, Carnaubawachs, Candilillawachs, Walrat sowie Mischungen der zuvor genannten Öl- bzw. Fettkomponenten.

Geeignete kosmetisch und pharmazeutisch verträgliche Öl- bzw. Fettkomponenten sind in Karl-Heinz Schrader, Grundlagen und Rezepturen der Kosmetika, 2. Auflage, Verlag Hüthig, Heidelberg, S. 319-355 beschrieben, worauf hier Bezug genommen wird.

Bei den erfindungsgemäßen kosmetischen Mitteln kann es sich um hautkosmetische, dermatologische oder haarkosmetische Mittel handeln.

Vorzugsweise liegen die erfindungsgemäßen Mittel in Form eines Gels, Schaums, Sprays, einer Salbe, Creme, Emulsion, Suspension, Lotion, Milch oder Paste vor. Gewünschtenfalls können auch Liposomen oder Mikrospären eingesetzt werden. Bevorzugte Formulierungen sind Gele, Schäume und Sprays. Die erfindungsgemäßen und erfindungsgemäß eingesetzten Copolymere A) weisen vorteilhafterweise eine hohe Verträglichkeit mit anderen kosmetischen und pharmazeutischen Inhaltsstoffen, insbesondere Haarpolymeren, auf. Sie eignen sich besonders zur Herstellung klarer, fester Gele.

Die erfindungsgemäßen kosmetisch oder pharmazeutisch aktiven Mittel können zusätzlich kosmetisch und/oder dermatologisch aktive Wirkstoffe sowie Hilfsstoffe enthalten.

Geeignete kosmetisch und/oder dermatologisch aktive Wirkstoffe sind z.B. färbende Wirkstoffe, Haut- und Haarpigmentierungsmittel, Tönungsmittel, Bräunungsmittel, Bleichmittel, Keratin-härtende Stoffe, antimikrobielle Wirkstoffe, Lichtfilterwirkstoffe, Repellentwirkstoffe, hyperemisierend wirkende Stoffe, keratolytisch und keratoplastisch wirkende Stoffe, Antischuppenwirkstoffe, Antiphlogistika, keratinisierend wirkende Stoffe, antioxidativ bzw. als Radikalfänger aktive Wirkstoffe, hautbefeuchtende oder -feuchthaltende Stoffe, rückfettende Wirkstoffe, antierythimatös oder antiallergisch aktive Wirkstoffe und Mischungen davon.

Künstlich hautbräunende Wirkstoffe, die geeignet sind, die Haut ohne natürliche oder künstliche Bestrahlung mit UV-Strahlen zu bräunen, sind z.B. Dihydroxyaceton, Alloxan und Walnussschalenextrakt. Geeignete Keratin-härtende Stoffe sind in der Regel Wirkstoffe, wie sie auch in Antitranspirantien eingesetzt werden, wie z.B. Kaliumaluminiumsulfat, Aluminiumhydroxychlorid, Aluminiumlactat, etc. Antimikrobielle Wirkstoffe werden eingesetzt, um Mikroorganismen zu zerstören bzw. ihr Wachstum zu hemmen und dienen somit sowohl als Konservierungsmittel als auch als desodorierend wirkender Stoff, welcher die Entstehung oder die Intensität von Körpergeruch vermindert. Dazu zählen z.B. übliche, dem Fachmann bekannte Konservierungsmittel, wie p-Hydroxybenzoesäureester, Imidazolidinyl-Harnstoff, Formaldehyd, Sorbinsäure, Benzoesäure, Salicylsäure, etc. Derartige desodorierend wirkende Stoffe sind z.B. Zinkricinoleat, Triclosan, Undecylensäurealkylolamide, Citronensäuretriethylester, Chlorhexidin etc. Geeignete Lichtfilterwirkstoffe sind Stoffe, die UV-Strahlen im UV-B- und/oder UV-A-Bereich absorbieren. Geeignete UV-Filter sind z.B. 2,4,6-Triaryl-1,3,5-triazine, bei denen die Arylgruppen jeweils wenigstens einen Substituenten tragen können, der vorzugsweise ausgewählt ist unter Hydroxy, Alkoxy, speziell Methoxy, Alkoxycarbonyl, speziell Methoxycarbonyl und Ethoxycarbonyl und Mischungen davon. Geeignet sind weiterhin p-Aminobenzoesäureester, Zimtsäureester, Benzophenone, Campherderivate sowie UV-Strahlen abhaltende Pigmente, wie Titandioxid, Talkum und Zinkoxid. Geeignete Repellentwirkstoffe sind Verbindungen, die in der Lage sind, bestimmte Tiere, insbesondere Insekten, vom Menschen abzuhalten oder zu vertreiben. Dazu gehört z.B. 2-Ethyl-1,3-hexandiol, N,N-Diethyl-m-toluamid etc. Geeignete hyperemisierend wirkende Stoffe, welche die Durchblutung der Haut anregen, sind z.B. ätherische Öle, wie Latschenkiefer, Lavendel, Rosmarin, Wacholderbeer, Rosskastanienextrakt, Birkenblätterextrakt, Heublumenextrakt, Ethylacetat, Campher, Menthol, Pfefferminzöl, Rosmarinextrakt, Eukalyptusöl, etc. Geeignete keratolytisch und keratoplastisch wirkende Stoffe sind z.B. Salicylsäure, Kalziumthioglykolat, Thioglykolsäure und ihre Salze, Schwefel, etc. Geeignete Antischuppen-Wirkstoffe sind z.B. Schwefel, Schwefelpolyethylenglykolsorbitanmonooleat, Schwefelricinolpolyethoxylat, Zinkpyrithion, Aluminiumpyrithion, etc. Geeignete Antiphlogistika, die Hautreizungen entgegenwirken, sind z.B. Allantoin, Bisabolol, Dragosantol, Kamillenextrakt, Panthenol, etc.

Die erfindungsgemäßen kosmetischen Mittel können als kosmetischen und/oder pharmazeutischen Wirkstoff (wie auch gegebenenfalls als Hilfsstoff) wenigstens ein kosmetisch oder pharmazeutisch akzeptables von Verbindungen der Komponente A) verschiedenes Polymer enthalten. Dazu zählen ganz allgemein nichtionische, anionische, kationische, amphotere und neutrale Polymere. Bevorzugt werden wasserlösliche nichtionische Polymere eingesetzt.

Bevorzugte Polymere sind nichtionische, wasserlösliche bzw. wasserdispergierbare Polymere oder Oligomere, wie Polyethylenimine und deren Salze, Polyvinylamine und deren Salze, Cellulosederivate, Polyvinylcaprolactam, z.B. Luviskol® Plus (BASF), oder Polyvinylpyrrolidon und deren Copolymere, insbesondere mit Vinylestern, wie Vinylacetat und/oder Vinylpropionat, z.B. Luviskol® VA 37 (BASF); Polyamide, z.B. auf Basis von Itaconsäure und aliphatischen Diaminen, wie sie z.B. in der DE-A-43 33 238 beschrieben sind. Bevorzugt sind weiterhin Homo- und Copolymere von Vinylacetat, wie sie z.B. in der US 5,632,977 beschrieben sind.

Bevorzugte Polymere sind auch nichtionische, siloxanhaltige, wasserlösliche oder -dispergierbare Polymere, z.B. Polyethersiloxane, wie Tegopren® (Fa. Goldschmidt) oder Belsil® (Fa. Wacker).

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, Polyasparaginsäure und deren Salze, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer® 100P), Copolymere aus Ethylacrylat und Methacrylsäure (z.B. Luviumer® MAE), Copolymere aus N-tert.-Butylacrylamid, Ethylacrylat, Acrylsäure (Ultrahold® 8, strong), Copolymere aus Vinylacetat, Crotonsäure und gegebenenfalls weitere Vinylester (z.B. Luviset® Marken), Maleinsäureanhydridcopolymere, gegebenenfalls mit Alkohol umgesetzt, anionische Polysiloxane, z.B. carboxyfunktionelle, t-Butylacrylat, Methacrylsäure (z.B. Luviskol® VBM), Copolymere von Acrylsäure und Methacrylsäure mit hydrophoben Monomeren, wie z.B. C₄-C₃₀-Alkylester der Meth(acrylsäure), C₄-C₃₀-Alkylvinylester, C₄-C₃₀-Alkylvinylether und Hyaluronsäure. Beispiele für anionische Polymere sind weiterhin Vinylacetat/Crotonsäure-Copolymere, wie sie beispielsweise unter den Bezeichnungen Resyn® (National Starch) und Gafset® (GAF) im Handel sind und Vinylpyrrolidon/Vinylacrylat-Copolymere, erhältlich beispielsweise unter dem Warenzeichen Luviflex® (BASF). Weitere geeignete Polymere sind das unter der Bezeichnung Luviflex® VBM-35 (BASF) erhältliche Vinylpyrrolidon/Acrylat-Terpolymer und Natriumsulfonat-haltige Polyamide oder Natriumsulfonat-haltige Polyester. Bevorzugt sind weiterhin Ethylacrylat/Methacrylsäure-Copolymere, wie z.B. Luviflex® Soft und Methacrylsäure/tert.-Butylacrylat-Pfropfcopolymere auf Polyalkylenoxid-haltige Silikonderivate, wie z.B. Luviflex® Silk.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidocopolymere (Polyquaternium-7) und Chitosan. Geeignete kationische (quaternisierte) Polymere sind auch Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen) und kationische Polymere auf pflanzlicher Basis, z.B. Guarpolymere, wie die Jaguar®-Marken der Fa. Rhodia.

Geeignete Polymere sind auch amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®).

Die Formulierungsgrundlage erfindungsgemäßer pharmazeutischer Mittel enthält bevorzugt pharmazeutisch akzeptable Hilfsstoffe. Pharmazeutisch akzeptabel sind die im Bereich der Pharmazie, der Lebensmitteltechnologie und angrenzenden Gebieten bekanntermaßen verwendbaren Hilfsstoffe, insbesondere die in einschlägigen Arzneibüchern (z.B. DAB Ph. Eur. BP NF) gelisteten sowie andere Hilfsstoffe, deren Eigenschaften einer physiologischen Anwendung nicht entgegenstehen.

Geeignete Hilfsstoffe können sein: Gleitmittel, Netzmittel, emulgierende und suspendierende Mittel, konservierende Mittel, Antioxidantien, Antireizstoffe, Chelatbildner, Emulsionsstabilisatoren, Filmbildner, Gelbildner, Geruchsmaskierungsmittel, Harze, Hydrokolloide, Lösemittel, Lösungsvermittler, Neutralisierungsmittel, Permeationsbeschleuniger, Pigmente, quaternäre Ammoniumverbindungen, Rückfettungs- und Überfettungsmittel, Salben-, Creme- oder Öl-Grundstoffe, Siliconderivate, Stabilisatoren, Sterilantien, Treibmittel, Trocknungsmittel, Trübungsmittel, Verdickungsmittel, Wachse, Weichmacher, Weißöle. Eine diesbezügliche Ausgestaltung beruht auf fachmännischem Wissen, wie sie beispielsweise in Fiedler, H. P. Lexikon der Hilfsstoffe für Pharmazie, Kosmetik und angrenzende Gebiete, 4. Aufl., Aulendorf: ECV-Editio-Kantor-Verlag, 1996, dargestellt sind.

Zur Herstellung der erfindungsgemäßen dermatologischen Mittel können die Wirkstoffe mit einem geeigneten Hilfsstoff (Exzipient) vermischt oder verdünnt werden. Exzipienten können feste, halb feste oder flüssige Materialien sein, die als Vehikel, Träger oder Medium für den Wirkstoff dienen können. Die Zumischung weiterer Hilfsstoffe erfolgt gewünschtenfalls in der dem Fachmann bekannten Weise.

Nach einer ersten bevorzugten Ausführungsform handelt es sich bei den erfindungsgemäßen Mitteln um ein Hautreinigungsmittel.

Bevorzugte Hautreinigungsmittel sind Seifen von flüssiger bis gelförmiger Konsistenz, wie Transparentseifen, Luxusseifen, Deoseifen, Cremeseifen, Babyseifen, Hautschutzseifen, Abrasiveseifen und Syndets, pasteuse Seifen, Schmierseifen und Waschpasten, flüssige Wasch-, Dusch- und Badepräparate, wie Waschlotionen, Duschbäder und -gele, Schaumbäder, Ölbäder und Scrub-Präparate.

Nach einer weiteren bevorzugten Ausführungsform handelt es sich bei den erfindungsgemäßen Mitteln um kosmetische Mittel zur Pflege und zum Schutz der Haut, Nagelpflegemittel oder Zubereitungen für die dekorative Kosmetik.

Besonders bevorzugt handelt es sich um Hautpflegemittel, Intimpflegemittel, Fußpflegemittel, Lichtschutzmittel, Sonnenschutzmittel, Repellents, Rasiermittel, Haarentfernungsmittel, Antiaknemittel, Make-ups, Mascara, Lippenstifte, Lidschatten, Kajalstifte, Eyeliner, Rouges und Augenbrauenstifte.

Bei den erfindungsgemäßen Hautpflegemitteln handelt es sich insbesondere um W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen.

Hautkosmetische und dermatologische Mittel auf Basis der zuvor beschriebenen Polymere A) zeigen vorteilhafte Wirkungen. Die Polymere können unter anderem zur Feuchthaltung und Konditionierung der Haut und zur Verbesserung des Hautgefühls beitragen. Die Polymere können auch als Verdicker in den Formulierungen wirken. Durch Zusatz der erfindungsgemäßen Polymere kann in bestimmten Formulierungen eine erhebliche Verbesserung der Hautverträglichkeit erreicht werden.

Hautkosmetische und dermatologische Mittel enthalten vorzugsweise wenigstens ein Copolymer A) in einem Anteil von etwa 0,001 bis 50 Gew.-%, vorzugsweise 0,01 bis 30 Gew.-%, ganz besonders bevorzugt 0,1 bis 20 Gew.-%, bezogen auf das Gesamtgewicht des Mittels.

Besonders Lichtschutzmittel auf Basis der Copolymere A) besitzen die Eigenschaft, die Verweilzeit der UV-absorbierenden Inhaltsstoffe im Vergleich zu gängigen Hilfsmitteln wie Polyvinylpyrrolidon zu erhöhen.

Je nach Anwendungsgebiet können die erfindungsgemäßen Mittel in einer zur Hautpflege geeigneten Form, wie z.B. als Creme, Schaum, Gel, Stift, Mousse, Milch, Spray (Pumpspray oder treibmittelhaltiger Spray) oder Lotion appliziert werden.

Die hautkosmetischen Zubereitungen können neben den Polymeren A) und geeigneten Trägern noch weitere in der Hautkosmetik übliche Wirkstoffe und Hilfsstoffe, wie zuvor beschrieben, enthalten. Dazu zählen vorzugsweise Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel, Collagen, Eiweißhydrolysate, Stabilisatoren, pH-Wert-Regulatoren, Farbstoffe, Salze, Verdicker, Gelbildner, Konsistenzgeber, Silicone, Feuchthaltemittel, Rückfetter und weitere übliche Additive.

Bevorzugte Öl- und Fettkomponenten der hautkosmetischen und dermatologischen Mittel sind die zuvor genannten mineralischen und synthetischen Öle, wie z.B. Paraffine, Siliconöle und aliphatische Kohlenwasserstoffe mit mehr als 8 Kohlenstoffatomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀-Fettsäuren, Wachsester, wie z.B. Jojobaöl, Fettalkohole, Vaseline, hydriertes Lanolin und azetyliertes Lanolin sowie Mischungen davon.

Man kann die erfindungsgemäßen Polymere auch mit herkömmlichen Polymeren abmischen, falls spezielle Eigenschaften eingestellt werden sollen.

Die Herstellung der kosmetischen oder dermatologischen Zubereitungen erfolgt nach üblichen, dem Fachmann bekannten Verfahren.

Bevorzugt liegen die kosmetischen und dermatologischen Mittel in Form von Emulsionen insbesondere als Wasser-in-Öl-(W/O)- oder Öl-in-Wasser(O/W)-Emulsionen vor. Es ist aber auch möglich, andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen, usw.

Die Herstellung von Emulsionen erfolgt nach bekannten Methoden. Die Emulsionen enthalten neben dem Copolymer A) in der Regel übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser. Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, dritter Teil, worauf hiermit ausdrücklich Bezug genommen wird.

Eine geeignete Emulsion, z.B. für eine Hautcreme etc., enthält im Allgemeinen eine wässrige Phase, die mittels eines geeigneten Emulgatorsystems in einer Öl- oder Fettphase emulgiert ist.

Der Anteil des Emulgatorsystems beträgt in diesem Emulsionstyp bevorzugt etwa 4 und 35 Gew.-%, bezogen auf das Gesamtgewicht der Emulsion. Vorzugsweise beträgt der Anteil der Fettphase etwa 20 bis 60 Gew.-%. Vorzugsweise beträgt der Anteil der wässrigen Phase etwa 20 und 70 %, jeweils bezogen auf das Gesamtgewicht der Emulsion. Bei den Emulgatoren handelt es sich um solche, die in diesem Emulsionstyp üblicherweise verwendet werden. Sie werden z.B. ausgewählt unter: C₁₂-C₁₈-Sorbitan-Fettsäureestern; Estern von Hydroxystearinsäure und C₁₂-C₃₀-Fettalkoholen; Mono- und Diestern von C₁₂-C₁₈-Fettsäuren und Glycerin oder Polyglycerin; Kondensaten von Ethylenoxid und Propylenglykolen; oxypropylenierten/oxyethylierten C₁₂-C₁₈-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolinalkohol.

Bevorzugte Fettkomponenten, welche in der Fettphase der Emulsionen enthalten sein können, sind: Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Siliconglykol-Copolymer, Fettsäuren und Fettalkohole enthalten.

Um die Retention von Ölen zu begünstigen, können neben den Polymeren A) auch Wachse verwendet werden, wie z.B. Carnaubawachs, Candilillawachs, Bienenwachs, mikrokristallines Wachs, Ozokeritwachs und Ca-, Mg- und Al-Oleate, -Myristate, -Linoleate und -Stearate.

Im Allgemeinen werden die Wasser-in-Öl-Emulsionen so hergestellt, dass die Fettphase und der Emulgator in einen Ansatzbehälter gegeben werden. Man erwärmt diesen bei einer Temperatur von etwa 50 bis 75°C, gibt dann die in Öl löslichen Wirkstoffe und/oder Hilfsstoffe zu und fügt unter Rühren Wasser hinzu, welches vorher etwa auf die gleiche Temperatur erwärmt wurde und worin man gegebenenfalls die wasserlöslichen Ingredienzien vorher gelöst hat. Man rührt, bis man eine Emulsion der gewünschten Feinheit erhält und lässt dann auf Raumtemperatur abkühlen, wobei gegebenenfalls weniger gerührt wird.

Nach einer weiteren bevorzugten Ausführungsform handelt es sich bei den erfindungsgemäßen Mitteln um ein Duschgel, eine Shampoo-Formulierung oder ein Badepräparat.

Solche Formulierungen enthalten wenigstens ein Polymer A) sowie üblicherweise anionische Tenside als Basistenside und amphotere und/oder nichtionische Tenside als Cotenside. Weitere geeignete Wirkstoffe und/oder Hilfsstoffe sind im Allgemeinen ausgewählt unter Lipiden, Parfümölen, Farbstoffen, organischen Säuren, Konservierungsstoffen und Antioxidantien sowie Verdickern/Gelbildnern, Hautkonditioniermitteln und Feuchthaltemitteln.

Diese Formulierungen enthalten vorzugsweise 2 bis 50 Gew.-%, bevorzugt 5 bis 40 Gew.-%, besonders bevorzugt 8 bis 30 Gew.-% Tenside, bezogen auf das Gesamtgewicht der Formulierung.

In den Wasch-, Dusch- und Badepräparaten können alle in Körperreinigungsmitteln üblicherweise eingesetzten anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Acylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid- oder Propylenoxideinheiten, bevorzugt 1 bis 3 Ethylenoxideinheiten im Molekül aufweisen. Dazu zählt z.B. Natriumlaurylsulfat.

Geeignete amphotere Tenside sind z.B. Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen, ethoxylierte Fettsäureamide, Alkylpolyglycoside oder Sorbitanetherester geeignet.

Außerdem können die Wasch-, Dusch- und Badepräparate übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), Guarhydroxypropyltrimethylammoniumchlorid (INCI: Hydroxylpropyl Guar Hydroxypropyltrimonium Chloride), Copolymere aus N-Vinylpyrrolidon und quaternisiertem N-Vinylimidazol (Polyquaterinium-16, -44, -46), Copolymere aus N-Vinylpyrrolidon/Dimethylaminoethylmethacrylat, quaternisiert mit Diethylsulfat (Polyquaternium-11) und andere.

Weiterhin können die Duschgel-/Shampoo-Formulierungen Verdicker, wie z.B. Kochsalz, PEG-55, Propylene Glykol Oleate, PEG-120 Methyl Glucose Dioleate und andere, sowie Konservierungsmittel, weitere Wirk- und Hilfsstoffe und Wasser enthalten.

Nach einer weiteren bevorzugten Ausführungsform handelt es sich bei den erfindungsgemäßen Mitteln um ein Haarbehandlungsmittel.

Erfindungsgemäße Haarbehandlungsmittel enthalten vorzugsweise wenigstens ein Copolymer A) in einer Menge im Bereich von etwa 0,1 bis 30 Gew.-%, bevorzugt 0,5 bis 20 Gew.-%, bezogen auf das Gesamtgewicht des Mittels.

Vorzugsweise liegen die erfindungsgemäßen Haarbehandlungsmittel in Form eines Haargels, Schaumfestigers, Haarmousses, Shampoos, Haarsprays oder Haarschaums vor. Haarsprays umfassen dabei sowohl Aerosolsprays als auch Pumpsprays ohne Treibgas. Haarschäume umfassen sowohl Aerosolschäume wie auch Pumpschäume ohne Treibgas.

Haarsprays und Haarschäume umfassen vorzugsweise überwiegend oder ausschließlich wasserlösliche oder wasserdispergierbare Komponenten. Sind die in den erfindungsgemäßen Haarsprays und Haarschäumen eingesetzten Verbindungen wasserdispergierbar, können sie in Form von wässrigen Mikrodispersionen mit Teilchendurchmessern von üblicherweise 1 bis 350 nm, bevorzugt 1 bis 250 nm, zur Anwendung gebracht werden. Die Feststoffgehalte dieser Präparate liegen dabei üblicherweise in einem Bereich von etwa 0,5 bis 20 Gew.-%. Diese Mikrodispersionen benötigen in der Regel keine Emulgatoren oder Tenside zu ihrer Stabilisierung.

Bevorzugte Haarbehandlungsmittel liegen in Form einer wässrigen Dispersion oder in Form einer alkoholischen oder wässrig-alkoholischen Lösung vor. Beispiele geeigneter Alkohole sind Ethanol, Propanol, Isopropanol und Mischungen davon.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polymere A) können auch in Kombination mit anderen Haarpolymeren in den Mitteln zur Anwendung kommen. Geeignete Polymere sind die zuvor beschriebenen.

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel in Form eines Gels enthält:
a) 0,1 bis 20 Gew.-%, bevorzugt 1 bis 10 Gew.-%, mindestens eines Polymers A), wie zuvor definiert,
b) 0 bis 40 Gew.-% wenigstens eines Trägers (Lösungsmittels), der ausgewählt ist unter C₂-C₅-Alkoholen, insbesondere Ethanol,
c) 0,01 bis 5 Gew.-%, bevorzugt 0,2 bis 3 Gew.-%, wenigstens eines Verdickers,
d) 0 bis 50 Gew.-% eines Treibmittels,
e) 0 bis 10 Gew.-%, bevorzugt 0,1 bis 3 Gew.-%, mindestens eines von a) verschiedenen Haarpolymers, vorzugsweise eines wasserlöslichen nichtionischen Polymers,
f) 0 bis 1 Gew.-% wenigstens eines Rückfetters, vorzugsweise ausgewählt unter Glycerin und Glycerinderivaten,
g) 0 bis 30 Gew.-% weiterer Wirk- und/oder Hilfsstoffe,
h) Wasser ad 100 Gew.-%.

Ein bevorzugtes Haarbehandlungsmittel in Form eines Sprays enthält:
a) 0,5 bis 20 Gew.-%, bevorzugt 1 bis 10 Gew.-%, mindestens eines Polymers A), wie zuvor definiert,
b) 50 bis 99,5 Gew.-%, bevorzugt 55 bis 99 Gew.-%, eines Trägers (Lösungsmittels), ausgewählt unter Wasser und wassermischbaren Lösungsmitteln, bevorzugt C₂-C₅-Alkoholen, insbesondere Ethanol, und Mischungen davon,
c) 0 bis 70 Gew.-%, bevorzugt 0,1 bis 50 Gew.-%, eines Treibmittels, vorzugsweise ausgewählt unter Dimethylether und Alkanen, wie z.B. Propan/Butan-Gemischen,
d) 0 bis 10 Gew.-%, bevorzugt 0,1 bis 5 Gew.-%, mindestens eines von a) verschiedenen Haarpolymers, vorzugsweise eines in Wasser löslichen oder dispergierbaren Polymers,
e) 0 bis 0,5 Gew.-%, bevorzugt 0,001 bis 2 Gew.-%, mindestens einer wasserlöslichen oder wasserdispergierbaren Siliconverbindung,
sowie gegebenenfalls weitere Wirkstoffe und/oder Hilfsstoffe, wie zuvor definiert.

Die Erfindung wird anhand der folgenden nicht einschränkenden Beispiele näher erläutert.

### Beispiele

### Beispiel 1: Lösungspolymerisation Methode A

| | | |
|---|---|---|
| Zulauf 1: | 400 g | Vinylpyrrolidon |
| | 400 g | Vinylcaprolactam |
| | | |
| Zulauf 2: | 75 g | Methacrylsäure |
| | 75 g | Ethylacrylat |
| | 170 g | Ethanol |
| | | |
| Zulauf 3: | 15 g | Wako 50 (2,2'-Azobis(2-amidinopropan)dihydrochlorid) |
| | 135 g | Wasser |
| | | |
| Zulauf 4: | 10 g | Wako 50 (2,2'-Azobis(2-amidinopropan)dihydrochlorid) |
| | 90 g | Wasser |

In einer Rührapparatur mit Rückflußkühler, Innenthermometer und drei separaten Zulaufvorrichtungen wurden 80 g von Zulauf 1,15 g von Zulauf 3 und 50 g Mowiol® 4/88 (Polyvinylalkohol Fa. Hoechst) in 1400 g Wasser vorgelegt und die Mischung unter Rühren auf ca. 60°C aufgeheizt. Nach dem Anpolymerisieren, erkennbar an einer beginnenden Viskositätserhöhung, wurde bei 60°C der Rest von Zulauf 1 innerhalb von 3 Stunden und der Rest von Zulauf 3 innerhalb von 4 Stunden zugegeben, wobei die Innentemperatur auf ca. 65°C erhöht wurde. Nach dem Ende der Zugabe wurde noch ca. 2 Stunden bei dieser Temperatur nachpolymerisiert. Die viskose Polymerlösung wurde durch Zugabe von 600 g Ethanol verdünnt. Die wässrig-ethanolische Lösung wurde unter Rühren auf eine Temperatur von ca. 80°C gebracht. Anschließend wurde der Zulauf 2 innerhalb von 2 Stunden und Zulauf 4 in 3 Stunden zudosiert und polymerisiert. Nach dem Ende der Zugabe wurde noch ca. 2 Stunden bei einer Temperatur von ca. 80°C nachpolymerisiert. Das Lösungsmittel wurde durch Wasserdampfdestillation entfernt. Die erhaltene Polymerlösung wurde bei einer Temperatur 95 bis 100°C solange gerührt, bis der Restmonomergehalt (Vinylpyrrolidon und Vinylcaprolactam) < 50 ppm war (ca. 1 Stunde). Man erhielt dabei eine ca. 30 %ige wässrige Dispersion. Pulverförmige Produkte können durch Sprühtrocknen oder Gefriertrocknen erhalten werden.

In Analogie wurden alle Produkte mit Ethylacrylat (Beispiele Nr. 6 und 20) polymerisiert.

### Beispiel 10: Lösungspolymerisation Methode B

| | | |
|---|---|---|
| Zulauf 1: | 300 g | Vinylpyrrolidon |
| | 300 g | Vinylcaprolactam |
| | | |
| Zulauf 2: | 20 g | Methacrylsäure |
| | 600 g | Acrylamid (50 %ige wässrige Lösung) |
| | 30 g | Dimethylaminoethylmethacrylat quaternisiert mit Dimethylsulfat |
| | | |
| Zulauf 3: | 15 g | Wako 50 (2,2'-Azobis(2-amidinopropan)dihydrochlorid) |
| | 135 g | Wasser |
| | | |
| Zulauf 4: | 10 g | Wako 50 (2,2'-Azobis(2-amidinopropan)dihydrochlorid) |
| | 90 g | Wasser |

In einer Rührapparatur mit Rückflußkühler, Innenthermometer und drei separaten Zulaufvorrichtungen wurden 60 g Zulauf 1,15 g Zulauf 3 und 50 g eines wasserlöslichen Polyetherurethans aus Polyethylenglycol und Hexamethylendiisocyanat in 1810 g Wasser vorgelegt und die Mischung unter Rühren auf 60°C aufgeheizt. Nach dem Anpolymerisieren, erkennbar an einer beginnenden Viskositätserhöhung, wurde bei 60°C der Rest von Zulauf 1 innerhalb von 3 Stunden und der Rest von Zulauf 3 innerhalb von 4 Stunden zugegeben, wobei die Innentemperatur auf ca. 65°C erhöht wurde. Nach dem Ende der Zugabe wurde noch ca. 2 Stunden bei dieser Temperatur nachpolymerisiert. Anschließend wird der Zulauf 2 innerhalb von zwei Stunden und Zulauf 4 in 3 Stunden zudosiert und bei 60°C polymerisiert. Nach dem Ende der Zugabe wurde noch ca. zwei Stunden bei einer Temperatur von ca. 80°C nachpolymerisiert. Die erhaltene Polymerlösung wird bei einer Temperatur 95 bis 100°C solange gerührt, bis der Restmonomergehalt (Vinylpyrrolidon und Vinylcaprolactam) < 50 ppm war (ca. 1 Stunde). Man erhielt eine ca. 30 %ige wässrige hellgelbe Polymerlösung.

Pulverförmige Produkte können durch Sprühtrocknen oder Gefriertrocknen erhalten werden.

In Analogie wurden alle Produkte ohne Ethylacrylat (Beispiele Nr. 2-5 und 7-19) polymerisiert.

**Tabelle 1:**

| Bsp. Nr. | Vorlage | | Schritt 1 | | | | Schritt 2 | | | | |
|---|---|---|---|---|---|---|---|---|---|---|---|
| | PVOH | P(EU) | VP | VCap | AM | VFA | MAS | AM | 350-MA | EA | Q-DMA EMA |
| A | | | - | - | - | - | - | | - | - | - |
| B | | | - | - | - | - | - | | - | - | - |
| 1 | 5 | - | 40 | 40 | - | - | 7,5 | - | - | 7,5 | - |
| 2 | 5 | 5 | 50 | - | 30 | - | 5 | 5 | - | - | - |
| 3 | 10 | - | 50 | - | - | 30 | 5 | 5 | - | - | - |
| 4 | - | 7 | 50 | 30 | - | - | 4 | - | - | - | 4 |
| 5 | - | 5 | 40 | 30 | - | - | 3 | - | 19 | - | 3 |
| 6 | 5 | - | 30 | 30 | - | - | 10 | - | 15 | 10 | - |
| 7 | 5 | 5 | 40 | - | 20 | - | 5 | 25 | - | - | - |
| 8 | 10 | - | 40 | - | - | 20 | 5 | - | 25 | - | - |
| 9 | - | 7 | 40 | 20 | - | - | 4 | 25 | - | - | 4 |
| 10 | - | 5 | 30 | 30 | - | - | 2 | 30 | - | - | 3 |
| 11 | 10 | - | 30 | - | - | - | 5 | - | 50 | - | 5 |
| 12 | 5 | 5 | 30 | 20 | - | - | 5 | 35 | - | - | - |
| 13 | - | 3 | 30 | 22 | - | - | 5 | - | 40 | - | - |
| 14 | - | 5 | 30 | 20 | - | - | 5 | 35 | - | - | 5 |
| 15 | - | 5 | 30 | 30 | - | - | 2 | 30 | - | - | 3 |
| 16 | 10 | - | 30 | 20 | 30 | - | 5 | 5 | - | - | - |
| 17 | 5 | - | 30 | 20 | 30 | - | 5 | 10 | - | - | - |
| 18 | 5 | - | 25 | 25 | 30 | - | 2,5 | - | 10 | - | 2,5 |
| 19 | - | 5 | 30 | 20 | 35 | - | 2,5 | - | 5 | - | 2,5 |
| 20 | - | 5 | 30 | 20 | 35 | - | 5 | - | - | 5 | 2,5 |

| | | | | | | | | | | | |
|---|---|---|---|---|---|---|---|---|---|---|---|
| alle Angaben in Gew.-Teilen PVOH: teilverseifter Polyvinylalkohol (Mowiol® 8-88, Fa. Clariant) P(EU): Polyetherurethan aus Polyethylenglycol (Mn=4000) und Hexamethylendiisocyanat VP: N-Vinylpyrrolidon VCap: N-Vinylcaprolactam AM: Acrylamid VFA: N-Vinylformamid MAS: Methacrylsäure AM: Acrylamid 350-MA: Polyethylenglycolmethacrylat (Mn=350) EA: Ethylacrylat Q-DMAEMA: Dimethylaminoethylmethacrylat-Dimethylsulfat | | | | | | | | | | | |

### Anwendungstechnische Eigenschaften

### Standardformulierung:

Aus 0,3 Gew.-% eines handelsüblichen Polyacrylsäureverdickers (Carbopol 940, Fa. BFGoodrich), neutralisiert mit Triethanolamin wird ein Gel formuliert, das bei Anwendung auf dem Haar im Wesentlichen keine Conditionier- oder Festigungswirkung zeigt. Die anwendungstechnischen Eigenschaften sind in Tabelle 2 wiedergegeben.

### Vergleichsbeispiele A, B:

Der Gelformulierung aus der Standardformulierung werden je 3 Gew.-% eines handelsüblichen Haarpolymers (Bsp. A: Polyvinylpyrrolidon, Bsp. B: Vinylpyrrolidon-Vinylacetat-Copolymer) zugegeben. Die anwendungstechnischen Eigenschaften sind in Tabelle 2 wiedergegeben. Die Produkte sind hinsichtlich ihrer Klebrigkeit noch verbesserungswürdig.

### Erfindungsgemäß:

Der Gelformulierung aus der Standardformulierung werden je 3 Gew.-% der Copolymere 1 bis 20 als haarkosmetischer Wirkstoff zugegeben. Es resultieren klare Formulierungen mit guter Conditionier- bzw. Festigerwirkung. Die anwendungstechnischen Eigenschaften sind ebenfalls in Tabelle-2 wiedergegeben.

### Bewertung:

A) Klarheit

| Note | Klarheit |
|---|---|
| 1 | klar |
| 1-2 | fast klar |
| 2 | handklar (klar beim Ausbilden eines dünnen Film auf der Hand) |
| 3 | leicht trüb |
| 4 | trüb |
| 5 | sehr trüb |

B) Viskosität

| Note | Viskosität |
|---|---|
| 1 | sehr fest |
| 1-2 | fest |
| 2 | mäßig fest |
| 3 | fließend |

C) Klebrigkeit
Die Klebrigkeit wurde bei einer relativen Luftfeuchtigkeit von 75 % und bei Umgebungstemperatur direkt an getrockneten Filmen der Gelformulierungen bestimmt.

| Note | Klebrigkeit |
|---|---|
| 1 | nicht klebrig |
| 2 | leicht klebrig |
| 3 | mäßig klebrig |
| 4 | klebrig |
| 5 | sehr klebrig |

**Tabelle 2:**

| Bsp. Nr. | | Klarheit | Viskosität | Klebrigkeit |
|---|---|---|---|---|
| A | 3% Luviskol K90 + 0,3 Carbopol 940/TEA | 2 | 1-2 | 4 |
| B | 3% Luviskol VA64 + 0,3 Carbopol 940/TEA | 1-2 | 2 | 3 |
| 1 | 3% Polymer + 0,3 Carbopol 940/TEA | 1 | 1-2 | 1-2 |
| 2 | | 2 | 1-2 | 1-2 |
| 3 | | 2 | 1-2 | 2 |
| 4 | | 1-2 | 1 | 1-2 |
| 5 | | 1-2 | 1-2 | 2-3 |
| 6 | | 2 | 2 | 2 |
| 7 | | 2 | 1-2 | 1-2 |
| 8 | | 2-3 | 1-2 | 2 |
| 9 | | 1 | 1 | 1-2 |
| 10 | | 1-2 | 1-2 | 1-2 |
| 11 | | 1-2 | 2 | 1-2 |
| 12 | | 2 | 1-2 | 1 |
| 13 | | 2-3 | 1-2 | 1-2 |
| 14 | | 1-2 | 1 | 1-2 |
| 15 | | 1 | 1 | 1 |
| 16 | | 1-2 | 2 | 1 |
| 17 | | 1 | 1-2 | 1-2 |
| 18 | | 1 | 1-2 | 1 |
| 19 | | 1-2 | 1 | 1 |
| 20 | | 1-2 | 2 | 1 |

| | | | | |
|---|---|---|---|---|
| A) Haargel: | | | | |

### Beispiele Nr. 1-20

### Haargel mit anionischem Verdicker:

| Phase 1: | [%] | CTFA |
|---|---|---|
| Polymer 1-20 (30 %ige wässrige Lösung) | 10,0 | |
| Glycerin | 0,2 | |
| D-Panthenol USP | 0,1 | Panthenol |
| Triethanolamin | 0,5 | |
| Wasser dest. | 39,2 | |
| Zusatzstoffe, Konservierungsmittel, lösliches ethoxyliertes Silikon, Parfüm | q.s. | |
| | | |

| Phase 2: | | |
|---|---|---|
| Carbopol 940 (1 %ige wässrige Suspension) | 30,0 | Carbomer |
| Wasser dest. | 20,0 | |

Zur Herstellung des Haargels werden die Komponenten eingewogen und homogenisiert, dabei wird Phase 2 in Phase 1 eingerührt.

### Beispiele Nr. 21-30

### Haargel mit einem weiteren Festigerpolymer und Verdicker:

| Phase 1: | | [%] | CTFA |
|---|---|---|---|
| Polymer 1, 4, 5, 9, 10, 11, 17-20 | | | |
| | (30 %ige wässrige Lösung) | 7,0 | |
| Luviskol VA 64 | | 1,0 | Vinylpyrrolidonvinylacetat-Copolymer |
| Uvinul MS 40 | | 0,2 | Benzophenon-4 |
| Glycerin | | 0,2 | |
| D-Panthenol USP | | 0,1 | Panthenol |
| Triethanolamin | | 0,5 | |
| Ethanol | | 10,0 | |
| Wasser dest. | | 31,0 | |
| Zusatzstoffe, Konservierungsmittel, lösliches ethoxyliertes Silikon, Parfüm | | q.s. | |
| | | | |

| Phase 2: | | | |
|---|---|---|---|
| Carbopol 940 (1 %ige wässrige Suspension) | | 30,0 | Carbomer |
| Wasser dest. | | 20,0 | |

Zur Herstellung des Haargels werden die Komponenten eingewogen und homogenisiert, dabei wird Phase 2 in Phase 1 eingerührt.

### B) Schaumfestiger:

### Beispiele Nr. 31-40

| Schaum Conditioner: | | [%] |
|---|---|---|
| Polymer 1-4, 15-20 | | |
| | (30 %ige wässrige Lösung) | 15,0 |
| Cremophor A 25 (Ceteareth 25/BASF) | | 0,2 |
| Comperlan KD (Coamide DEA/Henkel) | | 0,1 |
| Wasser | | 74,7 |
| Dimethylether | | 10,0 |
| Zusatzstoffe, Konservierungsmittel, Parfüm | | q.s. |

Zur Herstellung des Schaum Conditioners werden die Komponenten eingewogen und unter Rühren gelöst. Anschließend werden sie in einen Spender abgefüllt und das Treibgas zugesetzt.

### C) Haarspray:

### Beispiele Nr. 41-50

| VOC 80 Aerosol-Haarspray | | [%] |
|---|---|---|
| Polymer Nr. 1, 6, 9, 10, 12-15, 19, 20 | | |
| | (30 %ige wässrige Lösung) | 5,0 |
| Luviset PUR | | 1,50 |
| Wasser | | 13,50 |
| Dimethylether | | 40,00 |
| Ethanol | | 40,00 |
| Zusatzstoffe, Silikon, Parfüm, Entschäumer | | q.s. |

### Beispiele Nr. 51-60

| VOC 55 Aerosol-Haarspray | | [%] |
|---|---|---|
| Polymer Nr. 1, 6, 9, 10, 12-15, 19, 20 | | |
| | (30 %ige wässrige Lösung) | 7,0 |
| Luviset PUR | | 1,00 |
| Wasser | | 37,00 |
| Dimethylether | | 35,00 |
| Ethanol | | 20,00 |
| Zusatzstoffe, Silikon, Parfüm, Entschäumer | | q.s. |

### Beispiele Nr. 61-70

| VOC 55 Handpumpen-Spray | | [%] |
|---|---|---|
| Polymer Nr. 1, 6, 9, 10, 12-15, 19, 20 | | |
| | (30 %ige wässrige Lösung) | 10,0 |
| Wasser | | 35,00 |
| Ethanol | | 55,00 |
| Zusatzstoffe, Silikon, Parfüm, Entschäumer | | q.s. |

### D) Shampoo:

### Beispiele Nr. 71-80

| Conditioner Shampoo: | | | [%] |
|---|---|---|---|
| A) Texapon NSO 28 %ig | | | |
| | (Sodium Laureth Sulphate/Henkel) | | 50,0 |
| | Comperlan KD (Coamide DEA/Henkel) | | 1,0 |
| Polymer Nr. 4, 5, 9-11, 14, 15, 18-20 | | | |
| | | (30 %ige wässrige Lösung) | 3,0 |
| Wasser | | | 17,0 |
| Parfümöl | | | q.s. |
| | | | |
| B) Wasser | | | 27,5 |
| | Natriumchlorid | | 1,5 |
| | Konservierungsmittel | | q.s. |

Zur Herstellung des Shampoos werden die Komponenten der jeweiligen Phasen A) bzw B) eingewogen und unter Rühren homogenisiert. Dann wird Phase B) langsam in Phase A) eingerührt.

### Verwendung in der Hautkosmetik:

### Beispiele Nr. 81-100

| Standard O/W-Creme: | | | |
|---|---|---|---|
| | Ölphase: | [%] | CTFA Name |
| Cremophor A6 | | 3,5 | Ceteareth-6 (und) Stearyl Alkohol |
| Cremophor A25 | | 3,5 | Ceteareth-25 |
| Glycerinmonostearat s.e. | | 2,5 | Glyceryl Stearat |
| Paraffinöl | | 7,5 | Paraffin Oil |
| Cetylalkohol | | 2,5 | Cetyl Alkohol |
| Luvitol EHO | | 3,2 | Cetearyl Octanoat |
| Vitamin-E-acetat | | 1,0 | Tocopheryl Acetat |
| Nip-Nip | | 0,1 | Methyl- und Propyl-4-hydroxybenzoate (7:3) |
| | | | |

| | Wasser-Phase: | [%] | |
|---|---|---|---|
| Polymer Nr. 1-20 | | | |
| | (30 %ige wässrige Lösung) | 3,0 | |
| Wasser | | 74,6 | Water |
| 1,2-Propylenglycol | | 1,5 | Propylene Glycol |
| Germall II | | 0,1 | Imidazolidinyl-Urea |

Zur Herstellung der Creme werden die Komponenten der Ölphase und der Wasserphase jeweils getrennt eingewogen und unter Rühren bei einer Temperatur von 80°C homogenisiert. Dann wird die Wasserphase langsam in die Ölphase eingerührt. Unter Rühren läßt man langsam auf Raumtemperatur abkühlen.

### Beispiele Nr. 101-120

### Tageslotion:

| | Ölphase: | [%] | CTFA Name |
|---|---|---|---|
| Cremophor A6 | | 1,5 | Ceteareth-6 (und) Stearyl Alkohol |
| Cremophor A25 | | 1,5 | Ceteareth-25 |
| Glycerinmonostearat s.e. | | 5,0 | Glyceryl Stearat |
| Uvinul MS 40 | | 0,5 | Benzophenone-4 |
| Paraffinöl | | 3, 5 | Paraffin Oil |
| Cetylalkohol | | 0,5 | Cetyl Alkohol |
| Luvitol EHO | | 10,0 | Cetearyl Octanoate |
| D-Panthenol 50 P | | 3,0 | Panthenol und Propylenglycol |
| Vitamin-E-acetat | | 1,0 | Tocopheryl Acetat |
| Tegiloxan 100 | | 0,3 | Dimethicon |
| Nip-Nip | | 0,1 | Methyl- und Propyl-4-hydroxybenzoate (7:3) |
| | | | |

| | Wasser-Phase: | [%] | |
|---|---|---|---|
| Polymer Nr. 1-20 | | | |
| | (30 %ige wässrige Lösung) | 1,5 | |
| Wasser | | 70,0 | Water |
| 1,2-Propylenglycol | | 1,5 | Propylene Glycol |
| Germall II | | 0,1 | Imidazolidinyl-Urea |

Zur Herstellung der Creme werden die Komponenten der Ölphase und der Wasserphase jeweils getrennt eingewogen und unter Rühren bei einer Temperatur von 80°C homogenisiert. Dann wird die Wasserphase langsam in die Ölphase eingerührt. Unter Rühren läßt man langsam auf Raumtemperatur abkühlen.

## Patentansprüche

1. Kosmetisches oder pharmazeutisches Mittel enthaltend in einem kosmetisch akzeptablen Träger wenigstens ein wasserlösliches oder wasserdispergierbares Copolymer A), das keine Siliciumatom-haltigen Gruppen enthält und das durch zweistufige radikalische Copolymerisation eines Monomergemisches aus
a) 20 bis 99,9 Gew.-%, bezogen auf das Gesamtgewicht der Komponenten a) bis d), wenigstens eines N-Vinyllactams,
b) 0,1 bis 30 Gew.-%, bezogen auf das Gesamtgewicht der Komponenten a) bis d), wenigstens einer Verbindung mit einer radikalisch polymerisierbaren, α,β-ethylenisch ungesättigten Doppelbindung und mindestens einer anionogenen Gruppe pro Molekül,
c) 0,1 bis 65 Gew.-%, bezogen auf das Gesamtgewicht der Komponenten a) bis d), wenigstens einer radikalisch polymerisierbaren Verbindung, die ausgewählt ist unter
c1) offenkettigen, α,β-ethylenisch ungesättigten Verbindungen der allgemeinen Formel I 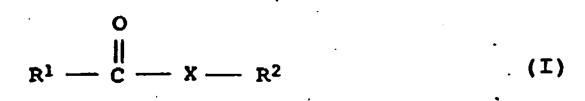 wobei
einer der Reste R¹ oder R² für eine Gruppe der Formel CH₂=CR³- mit R³ = H oder C₁-C₄-Alkyl steht und der andere für H, Alkyl, Hydroxyalkyl, Aminoalkyl, den N-Alkyl- und N,N-Dialkyl-Derivaten davon oder einen Polyetherrest mit mindestens 5 Alkylenoxideinheiten steht, und
X für O oder NR⁴ steht, wobei R⁴ für Wasserstoff, C₁-C₇-Alkyl oder einen Polyetherrest mit mindestens 5 Alkylenoxideinheiten steht,
c2) Verbindungen mit einer radikalisch polymerisierbaren, α,β-ethylenisch ungesättigten Doppelbindung und mindestens einer anionischen oder kationischen Gruppe pro Molekül,
d) 0 bis 15 Gew.-%, bezogen auf das Gesamtgewicht der Komponenten a) bis d), wenigstens eines von a) bis c) verschiedenen copolymerisierbaren Monomeren
in Gegenwart wenigstens einer wasserlöslichen Komponente e), die ausgewählt ist unter
e1) polyetherhaltigen Verbindungen, und/oder
e2) Polymeren, die mindestens 50 Gel.-% Wiederholungseinheiten aufweisen, die sich von Vinylalkohol ableiten,
erhältlich ist, wobei man:
i) die Monomere a) und gegebenenfalls wenigstens einen Teil der Monomere c) in Gegenwart wenigstens eines Teil der Komponente e) unter Erhalt eines ersten Polymerisats A1) polymerisiert, und
ii) die Monomere b) und falls vorhanden d) sowie die nicht bereits in Schritt i) eingesetzten Monomere c) und Verbindungen e) zugibt und unter Erhalt des Copolymers A) polymerisiert.

2. Mittel nach Anspruch 1, wobei die Komponente c) wenigstens eine Verbindung umfaßt, die ausgewählt ist unter N-Vinylamiden gesättigter Monocarbonsäuren, Amiden α,β-ethylenisch ungesättigter Monocarbonsäuren und deren N-Alkyl-, N-Polyalkylenglycol-, N,N-Dialkyl-, Aminoalkyl-, (N-Alkylamino)alkyl-, (N,N-Dialkylamino)alkyl- und N,N-Di(polyalkenylglycol)-Derivaten, Alkyl(meth)acrylaten, Polyetheracrylaten und Mischungen davon.

3. Mittel nach einem der vorhergehenden Ansprüche, wobei die Komponente e) wenigstens ein Polyetherurethan e1) umfasst.

4. Mittel nach einem der vorhergehenden Ansprüche, wobei das Copolymer A) erhältlich ist durch radikalische Copolymerisation von
20 bis 90 Gew.-% wenigstens eines N-Vinyllactams a),
1 bis 20 Gew.-% wenigstens einer Verbindung b) und
5 bis 65 Gew.-% wenigstens einer Verbindung c)
in Gegenwart wenigstens einer Komponente e).

5. Mittel nach Anspruch 4, wobei das Copolymerisat A) erhältlich ist durch radikalische Copolymerisation von
a) 20 bis 90 Gew.-% N-Vinylpyrrolidon und/oder N-Vinylcaprolactam,
b) 1 bis 20 Gew.-% (Meth)acrylsäure,
c) 5 bis 65 Gew.-% wenigstens einer Verbindung, die ausgewählt ist unter N-Vinylamiden gesättigter Monocarbonsäuren, Amiden α,β-ethylenisch ungesättigter Monocarbonsäuren, N,N-Dialkylaminoalkyl(meth)acrylaten, C₁-C₃-Alkyl(meth)acrylaten, Polyalkylenglycol(meth)acrylaten und Mischungen davon,
in Gegenwart wenigstens einer Komponente e).

6. Mittel nach einem der vorhergehenden Ansprüche, wobei der kosmetisch akzeptable Träger ausgewählt ist unter
i) Wasser,
ii) wassermischbaren organischen Lösungsmitteln, vorzugsweise C₁-C₄-Alkanolen,
iii) Treibgasen,
iv) Ölen, Fetten, Wachsen,
v) von iv) verschiedenen Estern von C₆-C₃₀-Monocarbonsäuren mit ein-, zwei- oder dreiwertigen Alkoholen,
vi) gesättigten acyclischen und cyclischen Kohlenwasserstoffen,
vii) Fettsäuren,
viii) Fettalkoholen
und Mischungen davon.

7. Mittel nach einem der vorhergehenden Ansprüche in Form eines Gel, Schaums, Sprays, einer Salbe, Creme, Emulsion, Suspension, Lotion, Milch oder Paste.

8. Mittel nach einem der vorhergehenden Ansprüche, wobei die Polymerisation in Schritt i) in Wasser und in Schritt ii) in einem Wasser/C₁-C₄-Alkanol-Gemisch erfolgt.

9. Mittel nach einem der vorhergehenden Ansprüche, wobei die Polymerisation in Schritt i) bei einem pH-Wert ≥6,5 und in Schritt ii) bei einem pH-Wert <6,5 erfolgt.

10. Copolymer A) wie in einem der Ansprüche 1 bis 5 definiert.

11. Verwendung eines Copolymers A), wie in einem der Ansprüche 1 bis 5 definiert, in Hautreinigungsmitteln, Mitteln zur Pflege und zum Schutz der Haut, Nagelpflegemitteln, Zubereitungen für die dekorative Kosmetik und Haarbehandlungsmitteln.

12. Verwendung nach Anspruch 11 in Haarbehandlungsmitteln als Festiger und/oder als Conditioner.

13. Verwendung nach Anspruch 12, wobei das Mittel in Form eines Haargels, Shampoos, Schaumfestigers, Haarwassers, Haarsprays oder Haarschaums vorliegt.

## Claims

1. A cosmetic or pharmaceutical composition comprising, in a cosmetically acceptable carrier, at least one water-soluble or water-dispersible copolymer A), which does not comprise any groups comprising a silicon atom and which is obtainable by two-stage free-radical copolymerization of a monomer mixture of
a) 20 to 99.9% by weight, based on the total weight of components a) to d), of at least one N-vinyllactam,
b) 0.1 to 30% by weight, based on the total weight of components a) to d), of at least one compound with a free-radically polymerizable, α,β-ethylenically unsaturated double bond and at least one anionogenic group per molecule,
c) 0.1 to 65% by weight, based on the total weight of components a) to d), of at least one free-radically polymerizable compound chosen from
c1) open-chain, α,β-ethylenically unsaturated compounds of the formula I 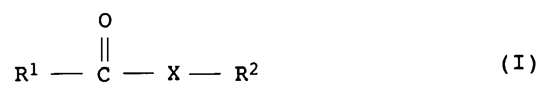 where
one of the radicals R¹ or R² is a group of the formula CH₂=CR³- where R³ = H or C₁-C₄-alkyl and the other is H, alkyl, hydroxyalkyl, aminoalkyl, the N-alkyl and N,N-dialkyl derivatives thereof or a polyether radical with at least 5 alkylene oxide units, and
X is O or NR⁴, where R⁴ is hydrogen, C₁-C₇-alkyl or a polyether radical having at least 5 alkylene oxide units,
c2) compounds with a free-radically polymerizable, α,β-ethylenically unsaturated double bond and at least one anionic or cationic group per molecule,
d) 0 to 15% by weight, based on the total weight of components a) to d), of at least one copolymerizable monomer which is different from a) to c)
in the presence of at least one water-soluble component e) which is chosen from
e1) polyether-containing compounds, and/or
e2) polymers which have at least 50% by weight of repeat units which are derived from vinyl alcohol,
where:
i) the monomers a) and if appropriate at least some of the monomers c) are polymerized in the presence of at least some of component e) to give a first polymer A1), and
ii) the monomers b) and if present d) and the monomers c) and compounds e) not already used in step i) are added and polymerized to give the copolymer A).

2. The composition according to claim 1, where component c) comprises at least one compound chosen from N-vinylamides of saturated monocarboxylic acids, amides of α,β-ethylenically unsaturated monocarboxylic acids and their N-alkyl, N-polyalkylene glycol, N,N-dialkyl, aminoalkyl, (N-alkylamino)alkyl, (N,N-dialkylamino)alkyl and N,N-di(polyalkenylene glycol) derivatives, alkyl(meth)acrylates, polyether acrylates and mixtures thereof.

3. The composition according to either of the preceding claims, where component e) comprises at least one polyether urethane e1).

4. The composition according to any of the preceding claims, where the copolymer A) is obtainable by free-radical copolymerization of
20 to 90% by weight of at least one N-vinyllactam a),
1 to 20% by weight of at least one compound b) and
5 to 65% by weight of at least one compound c)
in the presence of at least one component e).

5. The composition according to claim 4, where the copolymer A) is obtainable by free-radical copolymerization of
a) 20 to 90% by weight of N-vinylpyrrolidone and/or N-vinylcaprolactam,
b) 1 to 20% by weight of (meth)acrylic acid,
c) 5 to 65% by weight of at least one compound chosen from N-vinylamides of saturated monocarboxylic acids, amides of α,β-ethylenically unsaturated monocarboxylic acids, N,N-dialkylaminoalkyl (meth)acrylates, C₁-C₃-alkyl (meth)acrylates, polyalkylene glycol (meth)acrylates and mixtures thereof,
in the presence of at least one component e).

6. The composition according to any of the preceding claims, where the cosmetically acceptable carrier is chosen from
i) water,
ii) water-miscible organic solvents, preferably C₁-C₄-alkanols,
iii)propellant gases,
iv) oils, fats, waxes,
v) esters of C₆-C₃₀-monocarboxylic acids with mono-, di- or trihydric alcohols which are different from iv),
vi) saturated acyclic and cyclic hydrocarbons,
vii) fatty acids,
viii) fatty alcohols
and mixtures thereof.

7. The composition according to any of the preceding claims in the form of a gel, foam, spray, an ointment, cream, emulsion, suspension, lotion, milk or paste.

8. The composition according to any of the preceding claims, where the polymerization in step i) takes place in water and in step ii) in a water/C₁-C₄-alkanol mixture.

9. The composition according to any of the preceding claims, where the polymerization in step i) takes place at a pH of ≥ 6.5 and in step ii) at a pH of < 6.5.

10. A copolymer A) as defined in any of claims 1 to 5.

11. The use of a copolymer A) as defined in any of claims 1 to 5, in skin-cleansing compositions, compositions for caring and protecting the skin, nail care compositions, preparations for decorative cosmetics and hair-treatment compositions.

12. The use according to claim 11 in hair-treatment compositions as setting agents and/or as conditioners.

13. The use according to claim 12, where the composition is in the form of a hair gel, shampoo, setting foam, hair tonic, hairspray or hair foam.

## Revendications

1. Agent cosmétique ou pharmaceutique contenant, dans un support acceptable en cosmétique, au moins un copolymère A) soluble dans l'eau ou dispersible dans l'eau ne contenant aucun groupe contenant un atome de silicium et pouvant être obtenu grâce à la copolymérisation par voie radicalaire en deux étapes d'un mélange monomère issu de :
a) 20 à 99,9 % en poids, par rapport au poids total des composants a) à d), d'au moins un N-vinyllactame,
b) 0,1 à 30 % en poids, par rapport au poids total des composants a) à d), d'au moins un composé ayant une liaison double α,β-éthyléniquement insaturée polymérisable par voie radicalaire et d'au moins un groupe anionogène par molécule,
c) 0,1 à 65 % en poids, par rapport au poids total des composants a) à d), d'au moins un composé polymérisable par voie radicalaire, sélectionné parmi
c1) des composés à chaîne ouverte, α,β-éthyléniquement insaturés de formule générale I 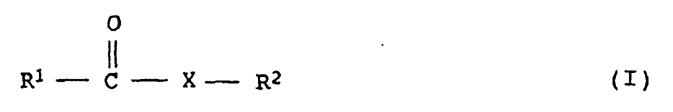 dans laquelle :
un des radicaux R¹ ou R² représente un groupe de formule CH₂ = CR³- avec R³ = H ou un groupe alkyle en C₁-C₄, et l'autre radical représente H, un groupe alkyle, hydroxyalkyle, aminoalkyle, des dérivés N-alkyliques et N,N-dialkyliques de ceux-ci ou un radical polyéther ayant au moins 5 unités oxyde d'alkylène, et
X représente O ou NR⁴, R⁴ représentant de l'hydrogène, un groupe alkyle en C₁-C₇ ou un radical polyéther ayant au moins 5 unités oxyde d'alkylène.
c2) des composés ayant une liaison double α,β-éthyléniquement insaturée polymérisable par voie radicalaire et au moins un groupe anionique ou cationique par molécule,
d) 0 à 15 % en poids, par rapport au poids total des composants a) à d), d'au moins un monomère copolymérisable différent de a) à c)
en présence d'au moins un composant e) soluble dans l'eau, sélectionné parmi :
e1) des composés contenant du polyéther, et/ou
e2) des polymères présentant au moins 50 % en poids d'unités de répétition et dérivant d'alcool vinylique,
dans lequel :
i) les monomères a) et éventuellement au moins une partie des monomères c) sont polymérisés en présence d'au moins une partie des composants e) avec obtention d'un premier polymère A1), et
ii) les monomères b) et, s'ils sont présents, d) ainsi que les monomères c) n'ayant pas déjà été utilisés dans l'étape i) et des composés e) sont ajoutés et sont polymérisés avec obtention du copolymère A.

2. Agent selon la revendication 1, dans lequel le composant c) comporte au moins un composé sélectionné parmi des N-vinylamides d'acides monocarboxyliques saturés, des amides d'acides monocarboxyliques α,β-éthyléniquement insaturés et leurs dérivés N-alkyliques, N-polyalkylèneglycoliques, N,N-dialkyliques, aminoalkyliques, (N-alkylamino)alkyliques, (N,N-dialkylamino)-alkyliques et N,N-di(polyalcénylglycoliques), des (méth)acrylates d'alkyle, des acrylates de polyéther et des mélanges de ceux-ci.

3. Agent selon l'une quelconque des revendications précédentes, dans lequel le composant e) comporte au moins un uréthanne de polyéther e1).

4. Agent selon l'une quelconque des revendications précédentes, dans lequel le copolymère A) peut être obtenu par copolymérisation par voie radicalaire de
20 à 90 % en poids d'au moins un N-vinyllactame a),
1 à 20 % en poids d'au moins un composé b) et
5 à 65 % en poids d'au moins un composé c)
en présence d'au moins un composant e).

5. Agent selon la revendication 4, dans lequel le copolymère A) peut être obtenu grâce à la copolymérisation par voie radicalaire de :
a) 20 à 90 % en poids de N-vinylpyrrolidone et/ou de N-vinylcaprolactame,
b) 1 à 20 % en poids d'acide (méth)acrylique,
c) 5 à 65 % en poids d'au moins un composé sélectionné parmi des N-vinylamides d'acides monocarboxyliques saturés, des amides d'acides monocarboxyliques α,β-éthyléniquement insaturés, des (méth)acrylates de N,N-dialkylaminoalkyle, des (méth)acrylates d'alkyle en C₁-C₃, des (méth)acrylates de polyalkylèneglycol et des mélanges de ceux-ci,
en présence d'au moins un composant e).

6. Agent selon l'une quelconque des revendications précédentes, dans lequel le support acceptable en cosmétique est sélectionné parmi :
i) de l'eau
ii) des solvants organiques miscibles dans l'eau, de préférence des alcanols en C₁-C₄,
iii) des gaz propulseurs,
iv) des huiles, des graisses, des cires,
v) des esters d'acides monocarboxyliques en C₆-C₃₀, différents de iv), avec des alcools monovalents, bivalents ou trivalents,
vi) des hydrocarbures saturés acycliques et cycliques,
vii) des acides gras,
viii) des alcools gras
et des mélanges de ceux-ci.

7. Agent selon l'une quelconque des revendications précédentes sous forme d'un gel, d'une mousse, d'un aérosol, d'une pommade, d'une crème, d'une émulsion, d'une suspension, d'une lotion, d'un lait ou d'une pâte.

8. Agent selon l'une quelconque des revendications précédentes, dans lequel la polymérisation a lieu dans l'étape i) dans l'eau et dans l'étape ii) dans un mélange eau/alcanol en C₁-C₄.

9. Agent selon l'une quelconque des revendications précédentes, dans lequel la polymérisation a lieu dans l'étape i) à une valeur de pH ≥ 6,5 et dans l'étape ii) à une valeur de pH < 6,5.

10. Copolymère A) tel que défini dans l'une des revendications 1 à 5.

11. Utilisation d'un copolymère A), tel que défini dans l'une des revendications 1 à 5, dans des produits de nettoyage de la peau, des produits pour le soin et la protection de la peau, des produits de soin des ongles, des préparations pour la cosmétique décorative et des produits de traitement capillaire.

12. Utilisation selon la revendication 11 dans des produits de traitement capillaire en tant que fortifiants et/ou en tant que après-shampooing.

13. Utilisation selon la revendication 12, dans laquelle l'agent est présent sous forme d'un gel pour les cheveux, d'un shampooing, d'une mousse fixante, d'une eau capillaire, d'un aérosol pour les cheveux ou d'une mousse pour les cheveux.
